# EUROPEAN PATENT SPECIFICATION

(11) **EP 2 809 785 B1**
(45) Date of publication and mention of the grant of the patent: **01.11.2017**
(21) Application number: 13703562.2
(22) Date of filing: 01.02.2013
(51) Int. Cl.: C12N 15/74, C12N 15/63

(54) **PROMOTERS FOR INCREASED PROTEIN EXPRESSION IN MENINGOCOCCUS**
PROMOTOREN FÜR ERHÖHTE PROTEINEXPRESSION BEI MENINGOKOKKEN
PROMOTEURS POUR UNE EXPRESSION PROTÉIQUE ACCRUE DANS LES MÉNINGOCOQUES

(30) Priority: 02.02.2012 US 201261594159 P
(43) Date of publication of application: 10.12.2014
(73) Proprietor: GlaxoSmithKline Biologicals SA, 1330 Rixensart (BE)
(72) Inventor: DELANY, Isabel, I-53100 Siena (IT); GUADAGNUOLO, Serafina, I-53100 Siena (IT)
(74) Representative: Courgeon, Antoine
(86) International application number: PCT/EP2013/052108
(87) International publication number: WO 2013/113917

(56) References cited:
- WO-A1-2013/033398
- WO-A2-01/09350
- WO-A2-2006/081259
- SAWAYA R ET AL: "Mutational analysis of the promoter region of the porA gene of Neisseria meningitidis", GENE, ELSEVIER, AMSTERDAM, NL, vol. 233, no. 1-2, 11 June 1999 (1999-06-11), pages 49-57, XP004173064, ISSN: 0378-1119, DOI: 10.1016/S0378-1119(99)00159-6
- A VAN DER ENDE ET AL: "Variable expression of class 1 outer membrane protein in Neisseria meningitidis is caused by variation in the spacing between the -10 and -35 regions of the promoter.", JOURNAL OF BACTERIOLOGY, vol. 177, no. 9, 1 May 1995 (1995-05-01), pages 2475-2480, XP055062651, ISSN: 0021-9193 cited in the application
- GORLA M C O ET AL: "Comparison of PorA VR types and porA promoter sequence from Neisseria meningitidis B isolated from non-immunised children and vaccine failures immunised with a serogroup B outer membrane protein vaccine", VACCINE, ELSEVIER LTD, GB, vol. 21, no. 21-22, 20 June 2003 (2003-06-20), pages 2871-2876, XP004429686, ISSN: 0264-410X, DOI: 10.1016/S0264-410X(03)00166-X
- ARHIN F F ET AL: "Sequencing of porA from clinical isolates of Neisseria meningitidis defines a subtyping scheme and its genetic regulation", CANADIAN JOURNAL OF MICROBIOLOGY, NRC RESEARCH PRESS, CA, vol. 44, no. 1, 1 January 1998 (1998-01-01), pages 56-63, XP009169600, ISSN: 0008-4166

## Description

### TECHNICAL FIELD

This invention is in the field of promoters for controlling transcription in meningococcal bacteria.

### BACKGROUND ART

*Neisseria meningitidis* is a cause of bacterial meningitis. One approach to meningococcal vaccination relies on outer membrane vesicles (OMVs), as used in the Novartis MENZB™ product and the Norwegian Institute of Public Health MENBVAC™ product, both of which are generated by detergent treatment of meningococci.

It is known to change the protein composition of meningococcal outer membranes, and thus to change the composition of OMVs. Knockout of undesirable genes and over-expression of desirable genes have both been described. References 1-3 report pre-clinical studies of an OMV vaccine in which fHbp (also known as GNA1870) is over-expressed (and this over-expression can be combined with knockout of LpxL1 [4]). Reference 5 reported a clinical study of five formulations of an OMV vaccine in which PorA & FrpB are knocked-out and Hsf & TbpA are over-expressed. Reference 6 reports a phase I clinical study of a native outer membrane vesicle vaccine prepared from bacteria having inactivated *synX* and *lpxL1* genes, over-expressed fHbp, two different PorA proteins and stabilised OpcA expression. Reference 7 reports a trivalent native outer membrane vesicle vaccine prepared from bacteria having inactivated *synX* and *lpxL1* genes (and, in two cases, inactivated *lgtA*)*,* two different PorA proteins, and over-expressed NadA or fHbp. Inactivation of genes such as *lpxL1* is important if vesicles will be generated by methods which do not remove LPS.

These changes in protein composition can be effected in various ways. For instance, the bacteria can be growing under iron-limiting conditions in order to stimulate the expression of certain proteins related to iron metabolism. Other techniques can involve engineering the bacteria. For instance, reference 8 suggests that strong promoters (such as the *porA, porB, lgtF,* or *hpuAB* promoters) might be used to up-regulate expression of protective outer membrane proteins, or that suppressive transcription control mechanisms might be removed. Reference 9 suggests that genes can be engineered to remove their phase variability. The *porA* promoter was used in reference 7 to over-express NadA, whereas fHbp was over-expressed using the *tac* promoter.

Sawaya R et al (Gene 1999 Jun 11; 233 (1-2): 49-57) performed mutational analysis of the promoter region of the porA gene of *Neisseria meningitides* and reported that promoter regions of porA from different clinical isolates were sequenced and were found to differ in the number of guanosine residues in a poly(G) track located upstream of the -10 region.

It is an object of the invention to provide further and improved ways of modifying protein expression in meningococci, and in particular to provide promoters for driving expression of genes of interest. Up-regulation can be used to increase the levels of useful proteins in OMVs.

### DISCLOSURE OF THE INVENTION

A first aspect of the invention uses modified *porA* promoters to drive transcription. The natural *porA* promoter, as used in examples 10-16 of reference 8, contains a poly-G sequence between its -35 and -10 regions, but this sequence can cause phase variation and so expression from the natural *porA* promoter can be unstable. The modified *porA* promoters of the invention are improved because they lack the wild-type poly-G sequence.

Thus, according to the first aspect, the invention provides a nucleic acid comprising a promoter having SEQ ID NO: 32, SEQ ID NO: 33 or SEQ ID NO: 34.

The promoter of the invention includes
(i) a -10 region from a meningococcal *porA* gene promoter and
(ii) a -35 region from a meningococcal *porA* gene promoter,
   wherein the -10 region and the -35 region are separated by an intervening (or spacer) sequence of 16 or 17 nucleotides, and wherein the intervening sequence includes a poly-guanidine sequence having no more than three consecutive guanidine nucleotides.

According to a second aspect, the invention provides a bacterial expression vector comprising a DNA sequence which includes a promoter according to the first aspect of the invention.

The nucleic acids of the invention can be present in a *meningococcus.* Therefore, the third aspect of the invention provides a *meningococcus* comprising a DNA sequence which includes a promoter according to the first aspect of the invention.

The promoters can drive expression of downstream protein-coding genes (in particular, genes encoding outer membrane proteins) to which they are operably linked, and the *meningococcus* can be used to prepare vaccines (in particular vesicle-based vaccines). Therefore, in a preferred embodiment, the promoter drives expression of an outer membrane protein.

### Promoters of the invention

The two essential sequences in bacterial promoters are the -10 region (also known as the Pribnow box) and the -35 region. These are separated by an intervening sequence, and between the -10 region and the transcription start site (+1) is a short non-transcribed upstream sequence.

The PorA promoter has been studied in detail. Reference 10 reports a 6-mer -35 region, followed by a 16-mer or 17-mer intervening sequence, then a 6-mer Pribnow box, then a 7-mer non-transcribed upstream sequence, followed by transcribed nucleotide +1. The start codon in the wild-type *porA* gene is at nucleotide +59 of the transcript, and this 59-mer spacing was confirmed in reference 8.

Promoters according to the invention include a -10 region from a meningococcal *porA* gene promoter, this is a 6-mer TATAAT *i.e.* a typical -10 region 6-mer.

Promoters according to the invention include a -35 region from a meningococcal *porA* gene promoter, this is a 6-mer, either TGGTTT or TTGACA.

The intervening sequence between the -35 region and the -10 region can be 16 or 17 nucleotides. The short non-transcribed upstream sequence is 7 nucleotides..

Thus a promoter according to the invention includes a 6-mer -35 region, a 16-mer or 17-mer intervening sequence, a 6-mer -10 region, and a 7-mer non-transcribed upstream sequence, giving 35 or 36 nucleotides of core promoter in total.

A promoter can, of course, continue upstream of the -35 region. Sequences upstream of the -35 region can be important for high-level expression from the promoter, of for regulation of expression from the promoters. For instance, in rRNA promoters sequences upstream of the core promoter called UP-elements can account for their exceptional strength increasing transcription as much as 300-fold [11,12]. These can be recognised by the α-subunit of the RNA polymerase core.

Promoters according to the invention contain the short non-transcribed upstream sequence TGAAGAC.

The intervening sequence of promoters according to the invention can be either TTTGCGAGGGAGGTGG (16-mer; SEQ ID NO: 27) or TTTTGCGAGGGAGGTGG (17-mer; SEQ ID NO: 28).

In the promoters according to the invention the poly-G sequence within the intervening sequence has no more than three consecutive guanidine residues.

When present in functioning DNA form within a bacterium these promoters are operably linked to a sequence which encodes a protein of interest, whose transcription is controlled by the promoter. The invention can be used to express any protein of interest, but the protein is preferably an outer membrane protein, preferably which can be retained in vesicles. Preferred outer membrane proteins are given below. Ideally the invention is not used to drive expression of a transcript which encodes a PorA outer membrane protein.

In a coding DNA sequence, downstream of a promoter of the invention the DNA includes a transcription start site, followed by a 5' untranslated region (typically including a Shine-Dalgarno sequence) and then a start codon for the encoded protein of interest.

It will be appreciated that discussions of promoter sequences herein refer to the sense strand. In double-stranded DNA, from which transcription takes place, the promoter has a complementary strand. Where two promoter elements are said to be separated by 'n' nucleotides, this is again a reference to the number of nucleotides in the sense strand; in double-stranded DNA the element will be separated by 'n' base pairs. If a promoter element is said to lack a guanidine residue, this is again a reference to the sense strand; in double-stranded DNA, for example, a guanidine-free promoter could include a guanidine residue, but only as a complementary base in the antisense strand for a cytosine residue in the sense strand. Although the invention is described by reference to the sense strand, the scope of the invention includes double-stranded nucleic acids including such sense strands, as well as single-stranded nucleic acids including such sense strands or including antisense forms of these sense strands (these antisense strands can, of course, be used to prepare the sense strands, by standard techniques).

### Vesicles

The modified PorA promoters of the invention are particularly useful when preparing meningococcal vesicles *i.e.* any proteoliposomic vesicle obtained by disruption of or blebbing from a meningococcal outer membrane to form vesicles therefrom that retain antigens from the outer membrane. Thus the term includes, for instance, OMVs (sometimes referred to as 'blebs'), microvesicles (MVs [13]) and 'native OMVs' ('NOMVs' [14]).

MVs and NOMVs are naturally-occurring membrane vesicles that form spontaneously during bacterial growth and are released into culture medium. MVs can be obtained by culturing *Neisseria* in broth culture medium, separating whole cells from the smaller MVs in the broth culture medium (*e.g.* by filtration or by low-speed centrifugation to pellet only the cells and not the smaller vesicles), and then collecting the MVs from the cell-depleted medium (*e.g.* by filtration, by differential precipitation or aggregation of MVs, by high-speed centrifugation to pellet the MVs). Strains for use in production of MVs can generally be selected on the basis of the amount of MVs produced in culture *e.g.* refs. 15 & 16 describe *Neisseria* with high MV production.

Another useful technique for spontaneous outer membrane vesicle production is to inactivate the *mltA* gene in a meningococcus, as disclosed in reference 17. These mutant bacteria release vesicles into their culture medium during growth.

OMVs are prepared artificially from bacteria, and may be prepared using detergent treatment (*e.g.* with deoxycholate), or by non-detergent means (*e.g.* see reference 18). Techniques for forming OMVs include treating bacteria with a bile acid salt detergent (*e.g.* salts of lithocholic acid, chenodeoxycholic acid, ursodeoxycholic acid, deoxycholic acid, cholic acid, ursocholic acid, *etc.,* with sodium deoxycholate [19 & 20] being preferred for treating *Neisseria*) at a pH sufficiently high not to precipitate the detergent [21]. Other techniques may be performed substantially in the absence of detergent [18] using techniques such as sonication, homogenisation, microfluidisation, cavitation, osmotic shock, grinding, French press, blending, *etc.* Methods using no or low detergent can retain useful antigens such as NspA [18]. Thus a method may use an OMV extraction buffer with about 0.5% deoxycholate or lower *e.g.* about 0.2%, about 0.1%, <0.05% or zero. A useful process for OMV preparation is described in reference 22 and involves ultrafiltration on crude OMVs, rather than instead of high speed centrifugation. The process may involve a step of ultracentrifugation after the ultrafiltration takes place.

A convenient way of purifying vesicles is the dual filtration method disclosed in reference 23.

Ideally the vesicles are those which form spontaneously during bacterial culture, rather than those which are released only after physical or chemical treatment of the bacteria. Growth of meningococci having an inactivated MltA is suitable way of producing vesicles in this way. Reference 46 discloses other ways of producing hyperblebbing strains. For spontaneously-released vesicles it is useful to use bacteria which do not produce intact LOS (detergent-extracted vesicles have reduced levels of potentially reactogenic LOS).

If lipo-oligosaccharide (LOS) is present in a vesicle it is possible to treat the vesicle so as to link its LOS and protein components ("intra-bleb" conjugation [47]).

The vesicles may lack LOS altogether, or they may lack hexa-acylated LOS *e.g.* LOS in the vesicles may have a reduced number of secondary acyl chains per LOS molecule [24]. For example, the vesicles may be from a strain which has a *lpxL1* deletion or mutation which results in production of a penta-acylated LOS [2,42]. LOS in a strain may lack a lacto-N-neotetraose epitope *e.g.* it may be an *lst* and/or *lgtB* knockout strain [5]. LOS may lack at least one wild-type primary O-linked fatty acid [25].The LOS may have no α chain. The LOS may comprise GlcNAc-Hep₂phosphoethanolamine-KDO₂-Lipid A [26].

The vesicles may include one, more than one, or (ideally) zero PorA serosubtypes. Modification of meningococcus to provide multi-PorA OMVs is known *e.g.* from reference 27, which discloses the construction of vesicles from strains modified to express six different PorA subtypes, and from reference 28. Conversely, modification to remove PorA is also known *e.g.* from reference 5.

The vesicles may be free from one of both of PorA and FrpB. Preferred vesicles are PorA-free.

The vesicles may lack capsular saccharide. For instance they may be derived from a strain that has one or more of the genes for capsule biosynthesis and/or export deactivated (*e.g. synX*)*.*

Mixtures of vesicles from different strains may be used. For instance, reference 29 discloses vaccine comprising multivalent meningococcal vesicle compositions, comprising a first vesicle derived from a meningococcal strain with a serosubtype prevalent in a country of use, and a second vesicle derived from a strain that need not have a serosubtype prevent in a country of use. Reference 30 also discloses useful combinations of different vesicles. A combination of vesicles from strains in each of the L2 and L3 immunotypes may be used.

### Over-expression

Promoters of the invention can be used to over-express a gene of interest in meningococcus. Where the gene encodes an outer membrane protein antigen, this over-expression can be used to provide a vesicle which advantageously retains that antigen. Such vesicles are discussed in more detail below. As a result of the over-expression, vesicles prepared from the modified meningococcus contain higher levels of the over-expressed antigen(s) than seen in a corresponding wild-type meningococcus. The increase in expression in the vesicles is usefully at least 10%, measured in mass of the relevant antigen per unit mass of vesicles, and is more usefully at least 20%, 30%, 40%, 50%, 75%, 100% or more.

Suitable recombinant modifications which can be used to over-express an antigen by using promoters of the invention include, but are not limited to: (i) promoter replacement; (ii) gene addition; and/or (iii) gene replacement. These three techniques can, if desired, be used in conjunction with (iv) repressor knockout.

In promoter replacement, the promoter which controls expression of the antigen's gene in a bacterium is replaced with a promoter of the invention in order to provide higher levels of expression.

In gene addition, a bacterium which already expresses the antigen receives a second copy of the relevant gene. This second copy can be integrated into the bacterial chromosome or can be on an episomal element such as a plasmid. The second copy can be expressed using a promoter of the invention. The effect of the gene addition is to increase the amount of expressed antigen. Where a plasmid is used, it is ideally a plasmid with a high copy number *e.g.* above 10, or even above 100.

In gene replacement, gene addition occurs but is accompanied by deletion of the existing copy of the gene. For instance, this approach was used in reference 3, where a bacterium's endogenous chromosomal *fHbp* gene was deleted and replaced by a plasmid-encoded copy (see also reference 31). Expression from the replacement copy, using a promoter of the invention, is higher than from the previous copy, thus leading to over-expression.

In some instances gene replacement occurs where gene addition is accompanied by the deletion of another gene. For instance, when the knocking out of an endogenous gene is required but is not the gene of interest that is being overexpressed by the promoter of the invention.

In some instances, more than one event of gene addition or gene replacement may occur such that expression from multiple copies of the gene of interest by promoters of the invention or combinations of overexpression of different genes of interest by promoters of the invention may take place.

Over-expression of at least one antigen will use a promoter of the invention, but these promoters can be used in conjunction with other techniques. For instance, the promoters can be used in conjunction with repressor knockout, in which a protein which represses expression of an antigen of interest is knocked out. This knockout means that the repression does not occur and the antigen of interest can be expressed at a higher level.

For instance, where NadA is over-expressed, the *nadA* gene can use a promoter of the invention, but in addition the gene encoding NadR (NMB1843) can be deleted. NadR a transcriptional repressor protein [32] which down-regulates or represses the NadA-encoding gene in all strains tested. Knockout of NadR results in constitutive expression of NadA. An alternative way to over-express NadA is to add 4-hydroxyphenylacetic to the culture medium. Thus promoters of the invention can be used as an over-expression strategy alone, or in combination with other approaches.

In a preferred embodiment a *meningococcus* according to the invention over-expresses NadA.

In a preferred embodiment a *meningococcus* according to the invention over-expresses NHBA.

In a preferred embodiment a *meningococcus* according to the invention over-expresses fHbp.

A *meningococcus* according to the invention may over-expresses both NHBA and NadA.

A *meningococcus* according to the invention may over-expresses both fHbp and NadA.

A *meningococcus* according to the invention may over-expresses both fHbp and NHBA.

A *meningococcus* according to the invention may over-expresses fHbp, NHBA and NadA.

In addition to over-expressing NHBA and/or NadA, a *meningococcus* according to the invention may over-express one or more further antigens. For instance, the bacterium may over-express one or more of: (a) NhhA; (b) TbpA; (c) HmbR; (d) TbpB; (e) NspA; (f) Cu,Zn-superoxide dismutase; (g) Omp85; (h) App; and/or (i) fHbp. Over-expression of NhhA is already reported in references 5 and 33. Over-expression of TbpA is already reported in references 5, 33 and 34. Over-expression of HmbR is already reported in reference 35. Over-expression of TbpB is already reported in reference 34. Over-expression of NspA is already reported in reference 36, in combination with *porA* and *cps* knockout. Over-expression of Cu,Zn-superoxide dismutase is already reported in reference 34. Over-expression of fHbp is already reported in references 1-3 & 31, and by a different approach (expressing a constitutively-active mutant FNR) in references 37 & 38. Where more than one antigen is over-expressed, at least one antigen will be over-expressed using a promoter of the invention, and more than one antigen may be over-expressed using a promoter of the invention.

In some embodiments a *meningococcus* over-expresses NHBA, NadA and fHbp. These three antigens are components of the "universal vaccine" disclosed in reference 39 or "4CMenB" [40,41].

An over-expressing modified strain will generally be isogenic with its parent strain, except for a genetic modification. As a result of the modification, expression of the antigen of interest in the modified strain is higher (under the same conditions) than in the parent strain.

### Bacteria

As mentioned above, vesicles are prepared from *meningococci* which over-express the relevant antigen(s) due to genetic modification, involving at least the use of a promoter of the invention. The invention also provides these *meningococcal* bacteria. In addition to including a promoter of the invention, the *meningococci* may include one or more further modifications. For instance, it can have a knockout of one or more of *lpxL1, lgtA, lgtB, porA, frpB, synX, mltA* and/or *lst.* For instance, reference 42 reports a NOMV vaccine prepared from bacteria having inactivated *synX, lpxL1,* and *lgtA* genes. Knockout of at least *lpxL1* and/or *synX* is particularly preferred.

The *meningococcal* bacterium may have low endotoxin levels, achieved by knockout of enzymes involved in LPS biosynthesis [43,44].

The *meningococcal* bacterium may be from any serogroup *e.g.* A, B, C, W135, or Y. It is preferably serogroup B.

The *meningococcal* bacterium may be of any serotype (*e.g.* 1, 2a, 2b, 4, 14, 15, 16, *etc.),* any serosubtype, and any immunotype (*e.g.* L1; L2; L3; L3,3,7; L10; *etc*.). Vesicles can usefully be prepared from strains having one of the following subtypes: P1.2; P1.2,5; P1.4; P1.5; P1.5,2; P1.5,c; P1.5c,10; P1.7,16; P1.7,16b; P1.7h,4; P1.9; P1.15; P1.9,15; P1.12,13; P1.13; P1.14; P1.21,16; P1.22,14.

The *meningococcal* bacterium may be from any suitable lineage, including hyperinvasive and hypervirulent lineages *e.g.* any of the following seven hypervirulent lineages: subgroup I; subgroup III; subgroup IV-1; ET-5 complex; ET-37 complex; A4 cluster; lineage 3. These lineages have been defined by multilocus enzyme electrophoresis (MLEE), but multilocus sequence typing (MLST) has also been used to classify meningococci [ref. 45] *e.g.* the ET-37 complex is the ST-11 complex by MLST, the ET-5 complex is ST-32 (ET-5), lineage 3 is ST-41/44, *etc.*

A bacterium may include one or more of the knockout and/or over-expression mutations disclosed in references 8, 36, 46 and 47. Suitable genes for modification include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB [8]; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB; and (d) CtrA, CtrB, CtrD, FrpB, OpA, OpC, PilC, PorB, SiaD, SynA, SynB, and/or SynC.

As mentioned above, in one preferred embodiment the *meningococcus* of the invention advantageously does not express an active MltA (GNA33), such that it spontaneously releases vesicles *e.g.* which contain antigens which are expressed using a promoter of the invention.

Ideally, the *meningococcus* does not express a native LPS *e.g.* it has a mutant or knockout of LpxL1 and/or of LgtB.

Where the *meningococcus* has a knockout, it is convenient to achieve this knockout by insertion of a sequence including a promoter of the invention controlling transcription of a protein of interest. For example, the genomic *synX* or *lpxL1* gene can be knocked out by inserting within it a sequence which includes a promoter of the invention which controls expression of an outer membrane protein such as fHbp. A single transformation event can thus be used to achieve two goals.

A *meningococcus* of the invention may include a selection marker *e.g.* an antibiotic resistance marker.

A *meningococcus* may have one or more, or all, of the following characteristics: (i) down-regulated or knocked-out LgtB and/or GalE to truncate the meningococcal LOS; (ii) up-regulated TbpA; (iii) up-regulated NhhA; (iv) up-regulated Omp85; (v) up-regulated LbpA; (vi) up-regulated NspA; (vii) knocked-out PorA; (viii) down-regulated or knocked-out FrpB; (ix) down-regulated or knocked-out Opa; (x) down-regulated or knocked-out Opc; (xii) deleted *cps* gene complex. A truncated LOS can be one that does not include a sialyl-lacto-N-neotetraose epitope *e.g.* it might be a galactose-deficient LOS. The LOS may have no α chain.

A *meningococcus* may have one or more, or all, of the following characteristics: (i) down-regulated or knocked-out LgtA; (ii) down-regulated or knocked-out LpxL1; (iii) down-regulated or knocked-out SynX; (iv) more than one PorA subtype, such as two different PorA subtypes; and/or (v) an up-regulated outer membrane antigen, such as NadA or fHbp.

### Strains production

Useful starting strains are in meningococcus serogroup B. Three useful starting meningococcal strains for preparing bacteria which over-express an antigen of interest are MC58, NZ98/254, and H44/76. MC58 has PorA serosubtype 1.7,16; NZ98/254 has serosubtype P1.7-2,4; and H44/76 has serosubtype 1.7,16. Other strains with these serosubtypes can also be used.

### Vectors etc.

The invention provides promoters as described above. These can be present in a bacterial chromosome, or in an extra-chromosomal or episomal nucleic acid *e.g.* within a plasmid. A bacterium can include 1 or more copies of the promoter of the invention. Where a bacterium includes 2 copies, these can be in the chromosome and/or episome(s).

The invention also provides a bacterial expression vector comprising a promoter of the invention.

These promoters within these vectors or bacteria can be 'orphan' promoters without any downstream transcribable sequence, but they will typically be operably linked to a sequence which is transcribed, and which is then translated to express a protein of interest.

### Antigens

### NHBA (Neisserial Heparin Binding Antigen)

NHBA [50] was included in the published genome sequence for meningococcal serogroup B strain MC58 [**Error! Bookmark not defined.**] as gene NMB2132 (GenBank accession number GI:7227388; SEQ ID NO: 9 herein). Sequences of NHBA from many strains have been published since then. For example, allelic forms of NHBA (referred to as protein '287') can be seen in Figures 5 and 15 of reference 48, and in example 13 and figure 21 of reference 49 (SEQ IDs 3179 to 3184 therein). Various immunogenic fragments of NHBA have also been reported.

NHBA antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 9; and/or (b) comprising a fragment of at least 'n' consecutive amino acids of SEQ ID NO: 9, wherein 'n' is 7 or more (*e.g.* 8, 10, 12, 14, 16, 18, 20, 25, 30, 35, 40, 50, 60, 70, 80, 90, 100, 150, 200, 250 or more). Ideally fragments of (b) comprise an epitope from SEQ ID NO: 9.

The most useful NHBA antigens can elicit antibodies which, after administration to a subject, can bind to a meningococcal polypeptide consisting of amino acid sequence SEQ ID NO: 9. Advantageous NHBA antigens can elicit bactericidal anti-meningococcal antibodies after administration to a subject.

Over-expression of NHBA has previously been achieved in various ways *e.g.* introduction of a NHBA gene under the control of an IPTG-inducible promoter [50].

### NadA (Neisserial adhesin A)

The NadA antigen was included in the published genome sequence for meningococcal serogroup B strain MC58 **[Error! Bookmark not defined.]** as gene NMB1994 (GenBank accession number GI:7227256; SEQ ID NO: 10 herein). The sequences of NadA antigen from many strains have been published since then, and the protein's activity as a Neisserial adhesin has been well documented. Various immunogenic fragments of NadA have also been reported.

NadA antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 10; and/or (b) comprising a fragment of at least 'n' consecutive amino acids of SEQ ID NO: 10, wherein 'n' is 7 or more (*e.g.* 8, 10, 12, 14, 16, 18, 20, 25, 30, 35, 40, 50, 60, 70, 80, 90, 100, 150, 200, 250 or more). Ideally fragments of (b) comprise an epitope from SEQ ID NO: 10.

The most useful NadA antigens can elicit antibodies which, after administration to a subject, can bind to a meningococcal polypeptide consisting of amino acid sequence SEQ ID NO: 10. Advantageous NadA antigens can elicit bactericidal anti-meningococcal antibodies after administration to a subject. SEQ ID NO: 6 is one such fragment.

### HmbR

The full-length HmbR sequence was included in the published genome sequence for meningococcal serogroup B strain MC58 **[Error! Bookmark not defined.]** as gene NMB1668 (SEQ ID NO: 7 herein). Reference 51 reports a HmbR sequence from a different strain (SEQ ID NO: 8 herein), and reference 35 reports a further sequence (SEQ ID NO: 19 herein). SEQ ID NOs: 7 and 8 differ in length by 1 amino acid and have 94.2% identity. SEQ ID NO: 19 is one amino acid shorter than SEQ ID NO: 7 and they have 99% identity (one insertion, seven differences) by CLUSTALW.

A polypeptide may comprises a full-length HmbR sequence, or a partial HmbR sequence. Thus a HmbR sequence may comprise an amino acid sequence having at least *i*% sequence identity to SEQ ID NO: 7, where the value of *i* is 50, 60, 70, 80, 90, 95, 99 or more. Alternatively a HmbR sequence may comprise a fragment of at least *j* consecutive amino acids from SEQ ID NO: 7, where the value of *j* is 7, 8, 10, 12, 14, 16, 18, 20, 25, 30, 35, 40, 50, 60, 70, 80, 90, 100, 150, 200, 250 or more. Alternatively a HmbR sequence may comprise an amino acid sequence (i) having at least *i*% sequence identity to SEQ ID NO: 7 and/or (ii) comprising a fragment of at least *j* consecutive amino acids from SEQ ID NO: 7.

Fragments of *j* amino acids may comprise an epitope from SEQ ID NO: 7. Such epitopes will usually comprise amino acids that are located on the surface of HmbR. Useful epitopes include those with amino acids involved in HmbR's binding to haemoglobin, as antibodies that bind to these epitopes can block the ability of a bacterium to bind to host haemoglobin. The topology of HmbR, and its critical functional residues, were investigated in reference 52. Fragments that retain a transmembrane sequence are useful, because they can be displayed on the bacterial surface *e.g.* in vesicles. Examples of long fragments of HmbR correspond to SEQ ID NOs: 21 and 22. If soluble HmbR is used, however, sequences omitting the transmembrane sequence, but typically retaining epitope(s) from the extracellular portion, can be used.

The most useful HmbR antigens can elicit antibodies which, after administration to a subject, can bind to a meningococcal polypeptide consisting of amino acid sequence SEQ ID NO: 7. Advantageous HmbR antigens can elicit bactericidal anti-meningococcal antibodies after administration to a subject.

### fHbp (factor H binding protein)

The fHbp antigen has been characterised in detail. It has also been known as protein '741' [SEQ IDs 2535 & 2536 in ref. 49], 'NMB1870', 'GNA1870' [53-55], 'P2086', 'LP2086' or 'ORF2086' [56-58]. It is naturally a lipoprotein and is expressed across all meningococcal serogroups. The structure of fHbp's C-terminal immunodominant domain ('fHbpC') has been determined by NMR [59]. This part of the protein forms an eight-stranded β-barrel, whose strands are connected by loops of variable lengths. The barrel is preceded by a short α-helix and by a flexible N-terminal tail.

The fHbp antigen falls into three distinct variants [60] and it has been found that serum raised against a given family is bactericidal within the same family, but is not active against strains which express one of the other two families *i.e.* there is intra-family cross-protection, but not inter-family cross-protection. A single fHbp variant may usefully include a fHbp from two or three of the variants. Thus it may be useful to use a combination of two or three different fHbps, selected from: (a) a first protein, comprising an amino acid sequence having at least *a*% sequence identity to SEQ ID NO: 1 and/or comprising an amino acid sequence consisting of a fragment of at least *x* contiguous amino acids from SEQ ID NO: 1; (b) a second protein, comprising an amino acid sequence having at least *b*% sequence identity to SEQ ID NO: 2 and/or comprising an amino acid sequence consisting of a fragment of at least *y* contiguous amino acids from SEQ ID NO: 2; and/or (c) a third protein, comprising an amino acid sequence having at least *c*% sequence identity to SEQ ID NO: 3 and/or comprising an amino acid sequence consisting of a fragment of at least *z* contiguous amino acids from SEQ ID NO: 3.

The value of *a* is at least 85 *e.g.* 86, 87, 88, 89, 90, 91, 92, 93, 94, 95, 96, 97, 98, 99, 99.5, or more. The value of *b* is at least 85 *e.g.* 86, 87, 88, 89, 90, 91, 92, 93, 94, 95, 96, 97, 98, 99, 99.5, or more. The value of *c* is at least 85 *e.g.* 86, 87, 88, 89, 90, 91, 92, 93, 94, 95, 96, 97, 98, 99, 99.5, or more. The values of *a, b* and *c* are not intrinsically related to each other.

The value of *x* is at least 7 *e.g.* 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value of *y* is at least 7 *e.g.* 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value of *z* is at least 7 *e.g.* 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The values of *x, y* and *z* are not intrinsically related to each other.

Where a single fHbp variant is used, a composition may include a polypeptide comprising (a) an amino acid sequence having at least *a*% sequence identity to SEQ ID NO: 1 and/or comprising an amino acid sequence consisting of a fragment of at least *x* contiguous amino acids from SEQ ID NO: 1; or (b) an amino acid sequence having at least *b*% sequence identity to SEQ ID NO: 2 and/or comprising an amino acid sequence consisting of a fragment of at least *y* contiguous amino acids from SEQ ID NO: 2; or (c) an amino acid sequence having at least *c*% sequence identity to SEQ ID NO: 3 and/or comprising an amino acid sequence consisting of a fragment of at least *z* contiguous amino acids from SEQ ID NO: 3.

Where a fHbp from two or three of the variants is used, a composition may include a combination of two or three different fHbps selected from: (a) a first polypeptide, comprising an amino acid sequence having at least *a*% sequence identity to SEQ ID NO: 1 and/or comprising an amino acid sequence consisting of a fragment of at least *x* contiguous amino acids from SEQ ID NO: 1; (b) a second polypeptide, comprising an amino acid sequence having at least *b*% sequence identity to SEQ ID NO: 2 and/or comprising an amino acid sequence consisting of a fragment of at least *y* contiguous amino acids from SEQ ID NO: 2; and/or (c) a third polypeptide, comprising an amino acid sequence having at least *c*% sequence identity to SEQ ID NO: 3 and/or comprising an amino acid sequence consisting of a fragment of at least z contiguous amino acids from SEQ ID NO: 3. The first, second and third polypeptides have different amino acid sequences.

Where the invention uses a fHbp from two of the variants, a composition can include both: (a) a first polypeptide, comprising an amino acid sequence having at least *a*% sequence identity to SEQ ID NO: 1 and/or comprising an amino acid sequence consisting of a fragment of at least *x* contiguous amino acids from SEQ ID NO: 1; and (b) a second polypeptide, comprising an amino acid sequence having at least *b*% sequence identity to SEQ ID NO: 2 and/or comprising an amino acid sequence consisting of a fragment of at least *y* contiguous amino acids from SEQ ID NO: 2. The first and second polypeptides have different amino acid sequences.

Where a fHbp from two of the variants is used, a composition can include both: (a) a first polypeptide, comprising an amino acid sequence having at least *a*% sequence identity to SEQ ID NO: 1 and/or comprising an amino acid sequence consisting of a fragment of at least *x* contiguous amino acids from SEQ ID NO: 1; (b) a second polypeptide, comprising an amino acid sequence having at least *c*% sequence identity to SEQ ID NO: 3 and/or comprising an amino acid sequence consisting of a fragment of at least *z* contiguous amino acids from SEQ ID NO: 3. The first and second polypeptides have different amino acid sequences.

Another useful fHbp is one of the modified forms disclosed, for example, in reference 61 *e.g.* comprising SEQ ID NO: 20 or 23 therefrom. These modified forms can elicit antibody responses which are broadly bactericidal against meningococci. SEQ ID NO: 77 in reference 61 is another useful fHbp sequence which can be used.

A useful modification of fHbp, including of the sequences mentioned above, is a mutation which reduces or removes the protein's affinity for factor H. For instance, references 62 & 63 disclose such mutations at residues Glu-283 and/or Glu-304 by their numbering (subtract 72 from this numbering scheme to match SEQ ID NO: 1 herein). Similarly, references 64 & 65 discloses mutation (*e.g.* to Ser) at position Arg-41 by their numbering (subtract 7 to match SEQ ID NO: 1) for variant 1 and at positions 80, 211, 218, 220, 222, and/or 236 by their numbering (subtract 7 to match SEQ ID NO: 2) for variant 2. Alignments will quickly reveal the corresponding residues for any fHbp sequence of interest. The Arg-41-Ser mutation in variant 1 sequences is particularly useful.

fHbp protein(s) in a OMV will usually be lipidated *e.g.* at a N-terminus cysteine. Alternatively, they may not be lipidated.

### NspA (Neisserial surface protein A)

The NspA antigen was included in the published genome sequence for meningococcal serogroup B strain MC58 [**Error! Bookmark not defined**.] as gene NMB0663 (GenBank accession number GI:7225888; SEQ ID NO: 11 herein). The antigen was previously known from references 66 & 67. The sequences of NspA antigen from many strains have been published since then. Various immunogenic fragments of NspA have also been reported.

Preferred NspA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 11; and/or (b) comprising a fragment of at least 'n' consecutive amino acids of SEQ ID NO: 11, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 11.

The most useful NspA antigens can elicit antibodies which, after administration to a subject, can bind to a meningococcal polypeptide consisting of amino acid sequence SEQ ID NO: 11. Advantageous NspA antigens can elicit bactericidal anti-meningococcal antibodies after administration to a subject.

### NhhA (Neisseria hia homologue)

The NhhA antigen was included in the published genome sequence for meningococcal serogroup B strain MC58 [**Error! Bookmark not defined.**] as gene NMB0992 (GenBank accession number GI:7226232; SEQ ID NO: 12 herein). The sequences of NhhA antigen from many strains have been published since *e.g.* refs 48 & 68, and various immunogenic fragments of NhhA have been reported. It is also known as Hsf.

NhhA antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 12; and/or (b) comprising a fragment of at least 'n' consecutive amino acids of SEQ ID NO: 12, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 12.

The most useful NhhA antigens can elicit antibodies which, after administration to a subject, can bind to a meningococcal polypeptide consisting of amino acid sequence SEQ ID NO: 12. Advantageous NhhA antigens can elicit bactericidal anti-meningococcal antibodies after administration to a subject.

### App (Adhesion and penetration protein)

The App antigen was included in the published genome sequence for meningococcal serogroup B strain MC58 [**Error! Bookmark not defined.**] as gene NMB1985 (GenBank accession number GI:7227246; SEQ ID NO: 13 herein). The sequences of App antigen from many strains have been published since then. It has also been known as 'ORF1' and 'Hap'. Various immunogenic fragments of App have also been reported.

App antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 13; and/or (b) comprising a fragment of at least 'n' consecutive amino acids of SEQ ID NO: 13, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 13.

The most useful App antigens can elicit antibodies which, after administration to a subject, can bind to a meningococcal polypeptide consisting of amino acid sequence SEQ ID NO: 13. Advantageous App antigens for can elicit bactericidal anti-meningococcal antibodies after administration to a subject.

### Omp85 (85kDa outer membrane protein)

The Omp85 antigen was included in the published genome sequence for meningococcal serogroup B strain MC58 [**Error! Bookmark not defined.**] as gene NMB0182 (GenBank accession number GI:7225401; SEQ ID NO: 14 herein). The sequences of Omp85 antigen from many strains have been published since then. Further information on Omp85 can be found in references 69 and 70. Various immunogenic fragments of Omp85 have also been reported.

Omp85 antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 14; and/or (b) comprising a fragment of at least 'n' consecutive amino acids of SEQ ID NO: 14, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 14.

The most useful Omp85 antigens can elicit antibodies which, after administration to a subject, can bind to a meningococcal polypeptide consisting of amino acid sequence SEQ ID NO: 14. Advantageous Omp85 antigens can elicit bactericidal anti-meningococcal antibodies after administration to a subject.

### TbpA

The TbpA antigen was included in the published genome sequence for meningococcal serogroup B strain MC58 [**Error! Bookmark not defined.**] as gene NMB0461 (GenBank accession number GI:7225687; SEQ ID NO: 23 herein). The sequences of TbpA from many strains have been published since then. Various immunogenic fragments of TbpA have also been reported.

TbpA antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 23; and/or (b) comprising a fragment of at least 'n' consecutive amino acids of SEQ ID NO: 23, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 23.

The most useful TbpA antigens can elicit antibodies which, after administration to a subject, can bind to a meningococcal polypeptide consisting of amino acid sequence SEQ ID NO: 23. Advantageous TbpA antigens can elicit bactericidal anti-meningococcal antibodies after administration to a subject.

### TbpB

The TbpB antigen was included in the published genome sequence for meningococcal serogroup B strain MC58 [**Error! Bookmark not defined.**] as gene NMB1398 (GenBank accession number GI:7225686; SEQ ID NO: 24 herein). The sequences of TbpB from many strains have been published since then. Various immunogenic fragments of TbpB have also been reported.

TbpB antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 24; and/or (b) comprising a fragment of at least 'n' consecutive amino acids of SEQ ID NO: 24, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 24.

The most useful TbpB antigens can elicit antibodies which, after administration to a subject, can bind to a meningococcal polypeptide consisting of amino acid sequence SEQ ID NO: 24. Advantageous TbpB antigens can elicit bactericidal anti-meningococcal antibodies after administration to a subject.

### Cu,Zn-superoxide dismutase

The Cu,Zn-superoxide dismutase antigen was included in the published genome sequence for meningococcal serogroup B strain MC58 [**Error! Bookmark not defined.**] as gene NMB1398 (GenBank accession number GI:7226637; SEQ ID NO: 25 herein). The sequences of Cu,Zn-superoxide dismutase from many strains have been published since then. Various immunogenic fragments of Cu,Zn-superoxide dismutase have also been reported.

Cu,Zn-superoxide dismutase antigens may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 25; and/or (b) comprising a fragment of at least 'n' consecutive amino acids of SEQ ID NO: 25, wherein 'n' is 7 or more (*e.g.* 8, 10, 12, 14, 16, 18, 20, 25, 30, 35, 40, 50, 60, 70, 80, 90, 100, 150, 200, 250 or more). Particularly suitable fragments of (b) comprise an epitope from SEQ ID NO: 25.

The most useful Cu,Zn-superoxide dismutase antigens can elicit antibodies which, after administration to a subject, can bind to a meningococcal polypeptide consisting of amino acid sequence SEQ ID NO: 25. Advantageous Cu,Zn-superoxide dismutase antigens can elicit bactericidal anti-meningococcal antibodies after administration to a subject.

### Pharmaceutical compositions

Vesicles are useful as active ingredients in immunogenic pharmaceutical compositions for administration to a patient. These will typically include a pharmaceutically acceptable carrier, and a thorough discussion of such carriers is available in reference 71.

Effective dosage volumes can be routinely established, but a typical human dose of the composition has a volume of about 0.5ml *e.g.* for intramuscular injection. The RIVM OMV-based vaccine was administered in a 0.5ml volume [72] by intramuscular injection to the thigh or upper arm. MeNZB™ is administered in a 0.5ml by intramuscular injection to the anterolateral thigh or the deltoid region of the arm. Similar doses may be used for other delivery routes *e.g.* an intranasal OMV-based vaccine for atomisation may have a volume of about 100µl or about 130µl per spray, with four sprays administered to give a total dose of about 0.5ml.

The pH of a pharmaceutical composition is usually between 6 and 8, *e.g.* between 6.5 and 7.5 (*e.g.* about 7). The pH of the RIVM OMV-based vaccine is 7.4 [73]. The RIVM OMV-based vaccine maintains pH by using a 10mM Tris/HCl buffer, and stable pH in compositions may be maintained by the use of a buffer *e.g.* a Tris buffer, a citrate buffer, phosphate buffer, or a histidine buffer. Thus pharmaceutical compositions will generally include a buffer.

Pharmaceutical compositions may be sterile and/or pyrogen-free. Compositions may be isotonic with respect to humans.

Compositions for administration to patients may be immunogenic, e.g. vaccine compositions. Vaccines may either be prophylactic (*i.e.* to prevent infection) or therapeutic (*i.e.* to treat infection), but will typically be prophylactic. Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The antigen content of immunological compositions will generally be expressed in terms of the amount of protein per dose. A dose of about 0.9 mg protein per ml is typical for OMV-based intranasal vaccines.Compositions may include an immunological adjuvant. Thus, for example, they may include an aluminium salt adjuvant or an oil-in-water emulsion (*e.g.* a squalene-in-water emulsion). Suitable aluminium salts include hydroxides (*e.g.* oxyhydroxides), phosphates (*e.g.* hydroxyphosphates, orthophosphates), (*e.g.* see chapters 8 & 9 of ref. 74), or mixtures thereof. The salts can take any suitable form (*e.g.* gel, crystalline, amorphous, *etc.),* with adsorption of antigen to the salt being ideal. The concentration of Al⁺⁺⁺ in a composition for administration to a patient may be less than 5mg/ml *e.g.* ≤4 mg/ml, ≤3 mg/ml, ≤2 mg/ml, ≤1 mg/ml, *etc.* An ideal range is between 0.3 and 1mg/ml, for example, a maximum of 0.85mg/dose. Aluminium hydroxide adjuvants are particularly suitable for use with meningococcal vaccines.

Meningococci affect various areas of the body and so pharmaceutical compositions may be prepared in various liquid forms. For example, the compositions may be prepared as injectables, either as solutions or suspensions. Pharmaceutical compositions may be prepared for pulmonary administration *e.g.* by an inhaler, using a fine spray. Pharmaceutical compositions may be prepared for nasal, aural or ocular administration *e.g.* as spray or drops. Injectables for intramuscular administration are typical.

Compositions may include an antimicrobial, particularly when packaged in multiple dose format. Antimicrobials such as thiomersal and 2-phenoxyethanol are commonly found in vaccines, but it is ideal to use either a mercury-free preservative or no preservative at all.

Compositions may comprise detergent *e.g.* a Tween (polysorbate), such as Tween 80. Detergents are generally present at low levels *e.g.* <0.01%.

Compositions may include residual detergent (*e.g.* deoxycholate) from OMV preparation. The amount of residual detergent is ideally less than 0.4µg (e.g. less than 0.2µg) for every µg of MenB protein.

If a composition includes LOS, the amount of LOS is ideally less than 0.e.g. less than 0.05µg) for every µg of protein.

Compositions may include sodium salts (*e.g.* sodium chloride) to give tonicity. A concentration of 10±2 mg/ml NaCl is typical *e.g.* about 9 mg/ml.

In addition to vesicles, immunogenic compositions can include soluble protein antigens. For instance, a useful composition can include vesicles in combination with one or more of (i) a NHBA antigen (ii) a NadA antigen and (iii) a fHbp antigen. For example, the vesicles can be mixed with the "4CMenB" vaccine (see above). For example, the composition can include fHbp both in soluble form and also in over-expressed vesicular form, with the two forms being different fHbp variants *e.g.* variant 1 in soluble form (as in 4CMenB) and variant 2 and/or 3 present in the surface of a vesicle, prepared from bacteria which express the variant 2 or 3 sequence.

Thus a composition may include: (i) vesicles, such as spontaneously-released vesicles which display a fHbp sequence; (ii) a soluble NHBA antigen, such as SEQ ID NO: 4; (iii) a soluble fHbp antigen, such as SEQ ID NO: 5; and (iv) a soluble NadA antigen, such as SEQ ID NO: 6.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references 75-81, *etc.*

The term "comprising" encompasses "including" as well as "consisting" *e.g.* a composition "comprising" X may consist exclusively of X or may include something additional *e.g.* X + Y.

The term "about" in relation to a numerical value *x* is optional and means, for example, *x*±10%.

Where the invention concerns an "epitope", this epitope may be a B-cell epitope and/or a T-cell epitope, but will usually be a B-cell epitope. Such epitopes can be identified empirically (*e.g.* using PEPSCAN [82,83] or similar methods), or they can be predicted (*e.g.* using the Jameson-Wolf antigenic index [84], matrix-based approaches [85], MAPITOPE [86], TEPITOPE [87,88], neural networks [89], OptiMer & EpiMer [90, 91], ADEPT [92], Tsites [93], hydrophilicity [94], antigenic index [95] or the methods disclosed in references 96-100, *etc.*). Epitopes are the parts of an antigen that are recognised by and bind to the antigen binding sites of antibodies or T-cell receptors, and they may also be referred to as "antigenic determinants".

References to a percentage sequence identity between two amino acid sequences means that, when aligned, that percentage of amino acids are the same in comparing the two sequences. This alignment and % homology or sequence identity can be determined using software programs known in the art, for example those described in section 7.7.18 of ref. 101. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 102.

The word "substantially" does not exclude "completely" *e.g.* a composition which is "substantially free" from Y may be completely free from Y. Where necessary, the word "substantially" may be omitted from the definition of the invention.

### BRIEF DESCRIPTION OF DRAWINGS

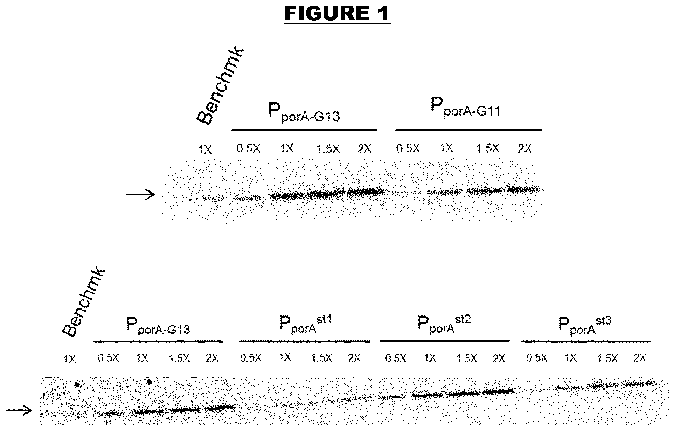

Figure 1 illustrates fHbp expression levels in the same background strain (NZ98/254) using five different promoters and the benchmark strain overexpressing the same fHbp allele in the same background [103,104]. A variant 2 allele of fHbp was overexpressed in the benchmark strain or from either 2 wildtype phase variants of the porA promoter with either 11 Gs or 13 Gs in the spacer tract or with the modified ST1, ST2 and ST3 porA promoters as indicated. Western blotting of equivalent quantities of total protein were loaded from 0.5x to 2x as indicated for each strain, and the arrow shows fHbp.

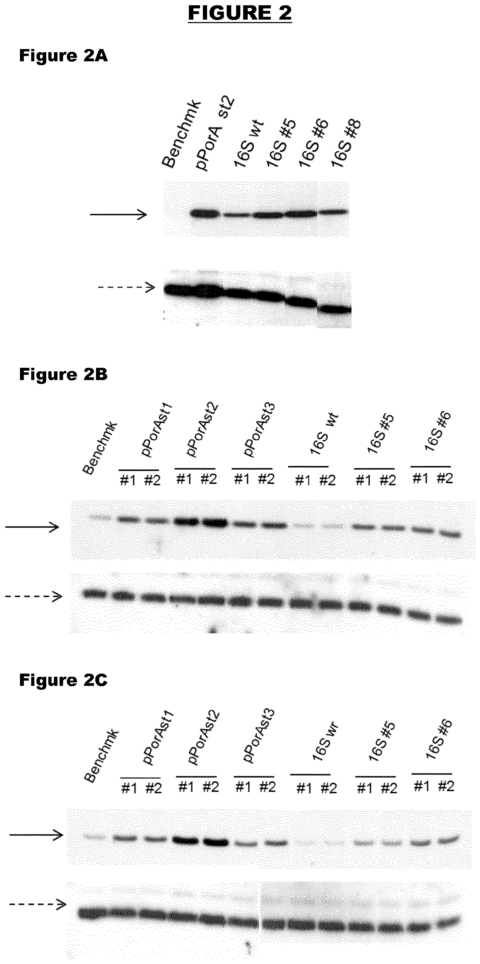

Figure 2 illustrates comparative analysis of fHbp expression from porA and 16S modified promoters. The NZ98/254 strain expressing fHBP variant 2 under the control of the indicated promoters was grown on plates (A) and in liquid to logarithmic (B) and stationary phase (C) and levels of fHBP expression were measured by western blot from duplicate cultures (#1, #2) or from the benchmark overexpression strain. The unbroken arrow shows fHbp, whereas the lower broken arrow shows a loading control.

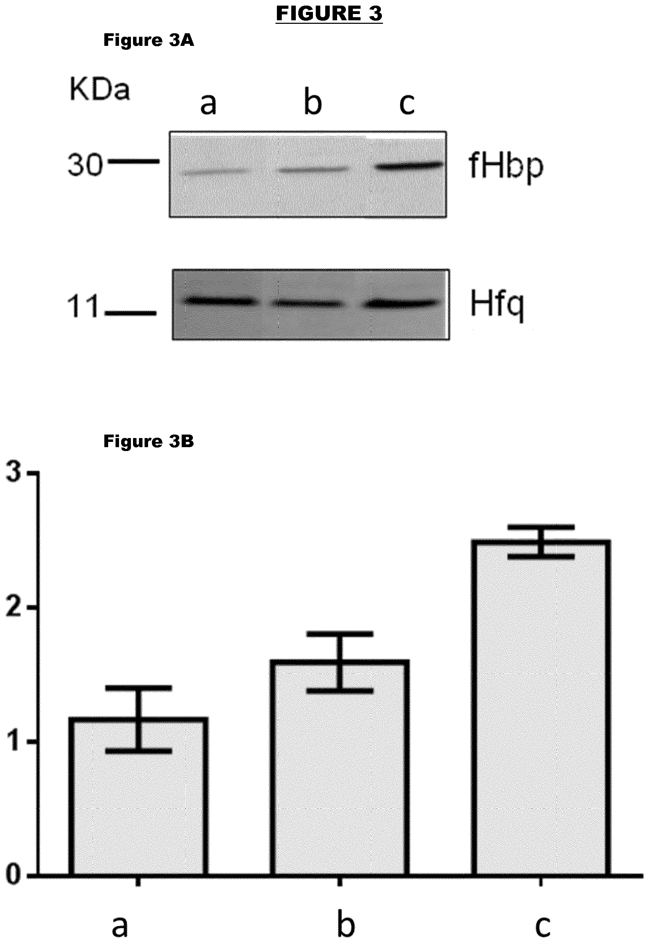

Figure 3 shows expression of fHbp from three strains: (a) a *ΔlpxL1* knockout; (b) a *ΔsynX* knockout; and (c) a *ΔpxL1ΔsynX* double knockout. In all cases the knocked out gene(s) was replaced by *fHbp* under the control of a modified PorA promoter. Expression of fHbp was assessed by western blot on crude cell extracts using anti-fHbp serum, and was compared to Hfq as a control. Figure 3A shows the western blot, and Figure 3B quantifies expression in arbitrary units.

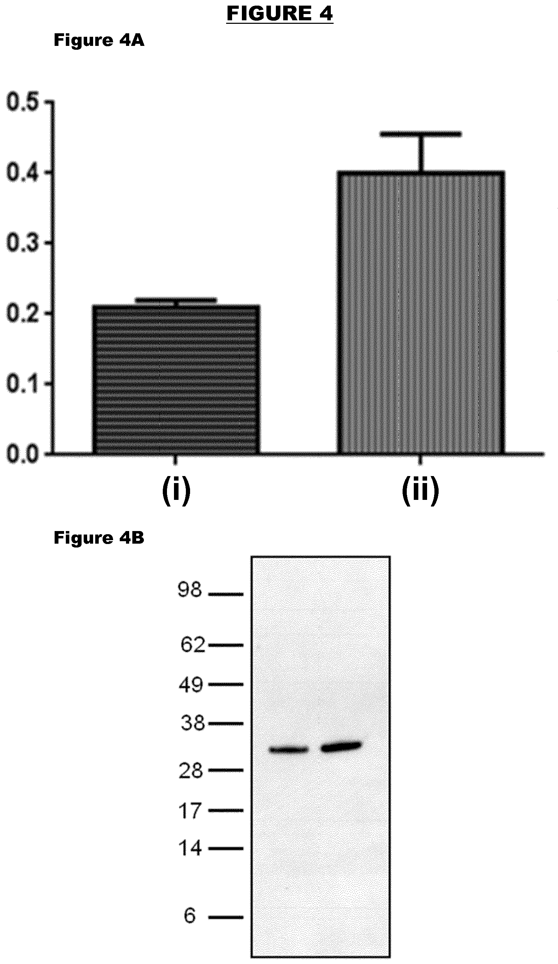

Figure 4 shows fHbp levels in vesicles isolated from (i) *ΔpxL1ΔsynX* and (ii) *ΔlpxL1ΔsynXΔmltA* knockout strains. Figure 4A shows yield in mg/OD, and Figure 4B is a western blot of 0.5µg vesicles.

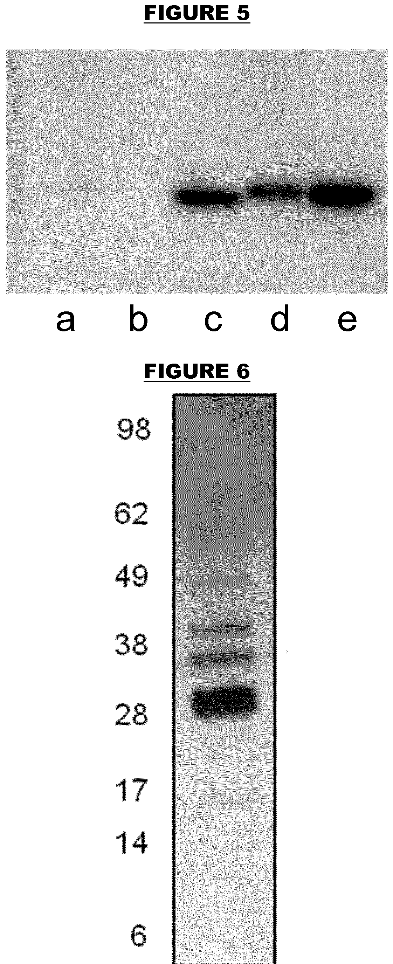

Figure 5 shows expression of fHbp in two strains that overexpress either variant 1 or variant 2 fHbp in distinct loci (lanes c & d) or a strain that overexpresses both variant 1 and variant 2 in respective loci (lane e). Lane a is the wild-type strain, and lane b is a *ΔfHbp* knockout of that strain.
Figure 6 shows silver-stained SDS-PAGE of vesicles purified from the triple knockout.

### MODES FOR CARRYING OUT THE INVENTION

### Modified PorA promoters

The sequences upstream of the transcription start site of two phase variants of the wild-type meningococcal *porA* promoter, containing 11 and 13 consecutive Gs in the spacer region, are as follows, with the -35 and -10 regions underlined:

| | |
|---|---|
| AAAAATGGTTTTTTGCGGGGGGGGGGGTATAATTGAAGAC | (SEQ ID NO: 31) |
| AAAAATGGTTTTTTGCGGGGGGGGGGGGGTATAATTGAAGAC | (SEQ ID NO: 40) |

The intervening sequence between the -35 and -10 regions includes a G₁₁ poly-G sequence which is connected to phase variation of PorA expression. To stabilise expression from this promoter, three modifications were tested (modifications in lower case):

| | |
|---|---|
| AAAAATGGTTTTTTGCGaGGGaGGtGGTATAATTGAAGAC | ("ST1"; SEQ ID NO: 32) |
| AAAAATtGacaTTTGCGaGGGaGGtGGTATAATTGAAGAC | ("ST2"; SEQ ID NO: 33) |
| AAAAtTGacaTTTTGCGaGGGaGGtGGTATAATTGAAGAC | ("ST3"; SEQ ID NO: 34) |

Thus in "ST1" the G₁₁ sequence was interrupted in three places, leaving no more than 3 consecutive G residues. In "ST2" the same intervening sequence was used, but the -35 region was substituted by a consensus -35 region TTGACA. In "ST3" an extra T residue was inserted immediately downstream of the -35 region.

These three promoters were used to drive expression of a fHbp sequence (fHbp variant 2) in the NZ98/258 strain. All three modified promoters resulted in high level overexpression of fHBP in the strain when compared to an equivalent overexpressing strain which has been reported in the literature [103,104]. The best expression was seen with ST2 (Figures 1 & 2).

### Modified rRNA promoters (not within the scope of the present invention)

In further experiments the 16S rRNA promoter was fused to the 5' UTR of the *porA* gene:

| | |
|---|---|
| ATATCTTGACAGGCGGAAGGAATACTTTATAATTCGCAAC... | (SEQ ID NO: 35) |

This promoter was subjected to mutation and three variants downstream of the -35 region were sequenced as follows:

| | |
|---|---|
| ATATCTTGACAGGCGGAAGGAATACTTTATAATTCGCAAC | ("wt"; SEQ ID NO: 35) |
| ATATCTTGACAGGCGGAAGGAATACTTTATATTCGCAAC... | ("#5"; SEQ ID NO: 36) |
| ATATCTTGACAGGCGGAAGGAATACTTTTAATTCGCAC... | ("#6"; SEQ ID NO: 37) |
| ATATCTTGACAGGCGGAAGGAAACTTTATAATTCGCAAC... | ("#8"; SEQ ID NO: 38) |

Thus in variants #5 and #6 the -10 sequence is a 5-mer, and variant #6 also lost a nucleotide between the -10 region and the transcription start site. In variant #8 the -10 region and its downstream sequence are the same as wt, but the intervening sequence between the -35 and -10 regions is one nucleotide shorter than wt.

### Expression of fHbp from modified promoters

These promoters were fused to the 5'UTR region of the *porA* gene and used to drive expression of the fHbp coding sequence. As seen in Figure 2, variants #5 and #6 showed the strongest expression, approaching levels seen with ST2.

The complete region spanning the wild-type promoter (SEQ ID NO: 35), extending downstream into the transcribed region and also upstream, was as follows (SEQ ID NO: 41; 358mer), with the -35 and -10 regions underlined, and nucleotide +1 (following reference 10) double-underlined:

Similarly, the surrounding sequences for variants #5 and #6 were as follows:

A *fHbp* gene under the control of a variant promoter was stably inserted into the chromosome of a meningococcus (strain NZ98/254) in place of the endogenous *lpxL1* and/or *synX* gene(s). Slightly higher expression levels were seen at the *synX* locus, and expression for the strain with both insertions *(ΔlpxL1ΔsynX* double knockout) was the sum of the expression from the individual loci (Figure 3).

Strains were also made in which two different fHbp variants (1 & 2) were expressed under the control of a modified PorA promoter. The co-expression of both variants has no significant negative effect on the expression from each distinct locus, and instead resulted in an additive effect (Figure 5).

Knockout of the endogenous *mltA* (GNA33) gene in the *ΔpxL1ΔsynX* double knockout further increased expression levels, and had no negative impact on the localisation of fHbp protein to the strain's vesicles. Figure 4 shows fHbp levels in vesicles isolated from these strains, showing (A) the yield of protein (mg) relative to the culture's optical density (OD), and (B) an anti-fHbp western blot against of 0.5µg of the vesicles. SDS-PAGE shows that the fHbp band in the triple knockout strain was one of the most abundant proteins in the vesicles, along with PorA and PorB (Figure 6).

### REFERENCES

[1] Koeberling et al. (2007) Vaccine 25:1912-20.
[2] Koeberling et al. (2008) J Infect Dis 198:262-70.
[3] Hou et al. (2005) J Infect Dis 192:580-90.
[4] WO2009/038889.
[5] Bonvehí et al. (2010) Clin Vacc Immunol 17:1460-6.
[6] Keiser et al. (2011) Vaccine 29:1413-20.
[7] Pinto et al. (2011) Vaccine 29:7752-8.
[8] WO01/09350.
[9] WO2004/015099.
[10] van der Ende et al. (1995) J Bacteriol 177:2475-80.
[11] Gourse et al. (1986) Cell 44:197-205.
[12] Hirvonen et al. (2001) J. Bacteriol. 183:6305-14.
[13] WO02/09643.
[14] Katial et al. (2002) Infect. Immun. 70:702-707.
[15] US patent 6,180,111.
[16] WO01/34642.
[17] WO2006/046143.
[18] WO2004/019977.
[19] European patent 0011243.
[20] Fredriksen et al. (1991) NIPH Ann. 14(2):67-80.
[21] WO01/91788.
[22] WO2005/004908.
[23] WO2011/036562.
[24] WO00/26384.
[25] US-6531131
[26] US-6645503
[27] Claassen et al. (1996) Vaccine 14:1001-8.
[28] de Kleijn et al. (2000) Vaccine 18:1456-66.
[29] WO03/105890.
[30] WO2006/024946
[31] WO2006/081259.
[32] Schielke et al. (2009) Mol Microbiol 72:1054-67.
[33] WO2004/014418.
[34] WO00/25811.
[35] WO2010/070453.
[36] WO02/09746.
[37] Oriente et al. (2010) J Bacteriol 192:691-701.
[38] US provisional patent application 61/247,428.
[39] Giuliani et al. (2006) Proc Natl Acad Sci U S A 103(29):10834-9.
[40] Donnelly et al. (2010) PNAS USA 107:19490-5.
[41] Kimura et al. (2010) Clin Vaccine Immunol. 2010 PMID: 21177912.
[42] Zollinger et al. (2010) Vaccine 28:5057-67.
[43] WO99/10497.
[44] Steeghs et al. (2001) The EMBO Journal 20:6937-6945.
[45] Maiden et al. (1998) PNAS USA 95:3140-3145.
[46] WO02/062378.
[47] WO2004/014417.
[48] WO00/66741.
[49] WO99/57280
[50] Serruto et al. (2010) PNAS USA 107:3770-5.
[51] US-5,698,438.
[52] Perkins-Balding et al. (2003) Microbiology 149:3423-35.
[53] Masignani et al. (2003) J Exp Med 197:789-799.
[54] Welsch et al. (2004) J Immunol 172:5605-15.
[55] Hou et al. (2005) J Infect Dis 192(4):580-90.
[56] WO03/063766.
[57] Fletcher et al. (2004) Infect Immun 72:2088-2100.
[58] Zhu et al. (2005) Infect Immun 73(10):6838-45.
[59] Cantini et al. (2006) J. Biol. Chem. 281:7220-7227
[60] WO2004/048404
[61] WO2009/104097.
[62] Schneider et al. (2009) Nature 458:890-3.
[63] WO2010/046715.
[64] Pajon et al. (2012) Infect Immun 80:2667-77.
[65] WO2011/126863.
[66] Martin et al. (1997) J Exp Med 185(7):1173-83.
[67] WO96/29412.
[68] WO01/55182.
[69] WO01/38350.
[70] WO00/23595.
*[75]* Methods In Enzymology (S. Colowick and N. Kaplan, eds., Academic Press, Inc.)
*[76]* Handbook of Experimental Immunology, Vols. I-IV (D.M. Weir and C.C. Blackwell, eds, 1986, Blackwell Scientific Publications)
[77] Sambrook et al. (2001) Molecular Cloning: A Laboratory Manual, 3rd edition (Cold Spring Harbor Laboratory Press).
*[78]* Handbook of Surface and Colloidal Chemistry (Birdi, K.S. ed., CRC Press, 1997)
[79] Ausubel et al. (eds) (2002) Short protocols in molecular biology, 5th edition (Current Protocols).
[80] Molecular Biology Techniques: An Intensive Laboratory Course, (Ream et al., eds., 1998, Academic Press)
[81] PCR (Introduction to Biotechniques Series), 2nd ed. (Newton & Graham eds., 1997, Springer Verlag)
[82] Geysen et al. (1984) PNAS USA 81:3998-4002.
[83] Carter (1994) Methods Mol Biol 36:207-23.
[84] Jameson, BA et al. 1988, CABIOS 4(1):181-186.
[85] Raddrizzani & Hammer (2000) Brief Bioinform 1(2):179-89.
[86] Bublil et al. (2007) Proteins 68(1):294-304.
[87] De Lalla et al. (1999) J. Immunol. 163:1725-29.
[88] Kwok et al. (2001) Trends Immunol 22:583-88.
[89] Brusic et al. (1998) Bioinformatics 14(2):121-30
[90] Meister et al. (1995) Vaccine 13(6):581-91.
[91] Roberts et al. (1996) AIDS Res Hum Retroviruses 12(7):593-610.
[92] Maksyutov & Zagrebelnaya (1993) Comput Appl Biosci 9(3):291-7.
[93] Feller & de la Cruz (1991) Nature 349(6311):720-1.
[94] Hopp (1993) Peptide Research 6:183-190.
[95] Welling et al. (1985) FEBS Lett. 188:215-218.
[96] Davenport et al. (1995) Immunogenetics 42:392-297.
[97] Tsurui & Takahashi (2007) J Pharmacol Sci. 105(4):299-316.
[98] Tong et al. (2007) Brief Bioinform. 8(2):96-108.
[99] Schirle et al. (2001) J Immunol Methods. 257(1-2):1-16.
[100] Chen et al. (2007) Amino Acids 33(3):423-8.
[101] Current Protocols in Molecular Biology (F.M. Ausubel et al., eds., 1987) Supplement 30
[102] Smith & Waterman (1981) Adv. Appl. Math. 2: 482-489.
[103] Koeberling et al. (2009) Clin Vaccin Immunol 16:156-62.
[104] Koeberling et al. (2011) Vaccine 29:4728-34.

## Claims

1. A nucleic acid comprising a promoter having SEQ ID NO: 32, SEQ ID NO: 33 or SEQ ID NO: 34.

2. A bacterial expression vector comprising a DNA sequence which includes the promoter of claim 1.

3. A *meningococcus* comprising a DNA sequence which includes the promoter of claim 1.

4. The *meningococcus* of claim 3, wherein the promoter drives expression of an outer membrane protein.

5. The *meningococcus* of claim 4, wherein the outer membrane protein is a fHbp.

6. The *meningococcus* of claim 4, wherein the outer membrane protein is NadA.

7. The *meningococcus* of claim 4, wherein the outer membrane protein is NHBA.

8. The *meningococcus* of any one of claims 4 to 7, wherein the bacterium does not express an active MltA and/or wherein the bacterium has a knockout of SynX and/or LpxL1.

9. The *meningococcus* of any one of claims 4 to 8, with serogroup B.

10. The *meningococcus* of claim 9 wherein the strain is MC58, NZ98/254 or H44/76.

## Patentansprüche

1. Nukleinsäure, welche einen Promotor umfasst, der SEQ ID NO: 32, SEQ ID NO: 33 oder SEQ ID NO: 34 hat.

2. Bakterieller Expressionsvektor, welcher eine DNA-Sequenz umfasst, die den Promotor gemäß Anspruch 1 einschließt.

3. *Meningococcus,* welcher eine DNA-Sequenz umfasst, die den Promotor gemäß Anspruch 1 einschließt.

4. *Meningococcus* gemäß Anspruch 3, wobei der Promotor die Expression eines Außenmembranproteins antreibt.

5. *Meningococcus* gemäß Anspruch 4, wobei das Außenmembranprotein ein fHbp ist.

6. *Meningococcus* gemäß Anspruch 4, wobei das Außenmembranprotein NadA ist.

7. *Meningococcus* gemäß Anspruch 4, wobei das Außenmembranprotein NHBA ist.

8. *Meningococcus* gemäß irgendeinem der Ansprüche 4 bis 7, wobei das Bakterium kein aktives MltA exprimiert und/oder wobei das Bakterium einen Knockout von SynX und/oder LpxL1 aufweist.

9. *Meningococcus* gemäß irgendeinem der Ansprüche 4 bis 8 mit Serogruppe B.

10. *Meningococcus* gemäß Anspruch 9, wobei der Stamm MC58, NZ98/254 oder H44/76 ist.

## Revendications

1. Acide nucléique comprenant un promoteur ayant une SEQ ID N° : 32, une SEQ ID N° : 33 ou une SEQ ID N° : 34.

2. Vecteur d'expression bactérien comprenant une séquence d'ADN qui comprend le promoteur selon la revendication 1.

3. Méningocoque comprenant une séquence d'ADN qui comprend le promoteur selon la revendication 1.

4. Méningocoque selon la revendication 3, dans lequel le promoteur dirige l'expression d'une protéine de la membrane externe.

5. Méningocoque selon la revendication 4, dans lequel la protéine de la membrane externe est une fHbp.

6. Méningocoque selon la revendication 4, dans lequel la protéine de la membrane externe est une NadA.

7. Méningocoque selon la revendication 4, dans lequel la protéine de la membrane externe est une NHBA.

8. Méningocoque selon l'une quelconque des revendications 4 à 7, dans lequel la bactérie n'exprime pas un MltA actif et/ou dans lequel la bactérie comporte une invalidation génique de SynX et/ou de LpxL1.

9. Méningocoque selon l'une quelconque des revendications 4 à 8, à sérogroupe B.

10. Méningocoque selon la revendication 9 dans lequel la souche est MC58, NZ98/254 ou H44/76.


### SEQUENCE LISTING

<110> NOVARTIS AG
<120> PROMOTERS FOR INCREASED PROTEIN EXPRESSION IN MENINGOCOCCUS
<130> P061061WO
<140> PCT/_2013/_
   <141> 2013-02-01
<150> US 61/594,159
   <151> 2012-02-02
<160> 43
<170> SeqWin2010, version 1.0
<210> 1
   <211> 248
   <212> PRT
   <213> Neisseria meningitidis
<400> 1
   Val Ala Ala Asp Ile Gly Ala Gly Leu Ala Asp Ala Leu Thr Ala Pro
   1               5                   10                  15
   Leu Asp His Lys Asp Lys Gly Leu Gln Ser Leu Thr Leu Asp Gln Ser
               20                  25                  30
   Val Arg Lys Asn Glu Lys Leu Lys Leu Ala Ala Gln Gly Ala Glu Lys
           35                  40                  45
   Thr Tyr Gly Asn Gly Asp Ser Leu Asn Thr Gly Lys Leu Lys Asn Asp
       50                  55                  60
   Lys Val Ser Arg Phe Asp Phe Ile Arg Gln Ile Glu Val Asp Gly Gln
   65                  70                  75                  80
   Leu Ile Thr Leu Glu Ser Gly Glu Phe Gln Val Tyr Lys Gln Ser His
                   85                  90                  95
   Ser Ala Leu Thr Ala Phe Gln Thr Glu Gln Ile Gln Asp Ser Glu His
               100                 105                 110
   Ser Gly Lys Met Val Ala Lys Arg Gln Phe Arg Ile Gly Asp Ile Ala
           115                 120                 125
   Gly Glu His Thr Ser Phe Asp Lys Leu Pro Glu Gly Gly Arg Ala Thr
       130                 135                 140
   Tyr Arg Gly Thr Ala Phe Gly Ser Asp Asp Ala Gly Gly Lys Leu Thr
   145                 150                 155                 160
   Tyr Thr Ile Asp Phe Ala Ala Lys Gln Gly Asn Gly Lys Ile Glu His
                   165                 170                 175
   Leu Lys Ser Pro Glu Leu Asn Val Asp Leu Ala Ala Ala Asp Ile Lys
               180                 185                 190
   Pro Asp Gly Lys Arg His Ala Val Ile Ser Gly Ser Val Leu Tyr Asn
           195                 200                 205
   Gln Ala Glu Lys Gly Ser Tyr Ser Leu Gly Ile Phe Gly Gly Lys Ala
       210                 215                 220
   Gln Glu Val Ala Gly Ser Ala Glu Val Lys Thr Val Asn Gly Ile Arg
   225                 230                 235                 240
   His Ile Gly Leu Ala Ala Lys Gln
                   245
<210> 2
   <211> 247
   <212> PRT
   <213> Neisseria meningitidis
<400> 2
   Val Ala Ala Asp Ile Gly Ala Gly Leu Ala Asp Ala Leu Thr Ala Pro
   1               5                   10                  15
   Leu Asp His Lys Asp Lys Ser Leu Gln Ser Leu Thr Leu Asp Gln Ser
               20                  25                  30
   Val Arg Lys Asn Glu Lys Leu Lys Leu Ala Ala Gln Gly Ala Glu Lys
           35                  40                  45
   Thr Tyr Gly Asn Gly Asp Ser Leu Asn Thr Gly Lys Leu Lys Asn Asp
       50                  55                  60
   Lys Val Ser Arg Phe Asp Phe Ile Arg Gln Ile Glu Val Asp Gly Gln
   65                  70                  75                  80
   Leu Ile Thr Leu Glu Ser Gly Glu Phe Gln Ile Tyr Lys Gln Asp His
                   85                  90                  95
   Ser Ala Val Val Ala Leu Gln Ile Glu Lys Ile Asn Asn Pro Asp Lys
               100                 105                 110
   Ile Asp Ser Leu Ile Asn Gln Arg Ser Phe Leu Val Ser Gly Leu Gly
           115                 120                 125
   Gly Glu His Thr Ala Phe Asn Gln Leu Pro Asp Gly Lys Ala Glu Tyr
       130                 135                 140
   His Gly Lys Ala Phe Ser Ser Asp Asp Ala Gly Gly Lys Leu Thr Tyr
   145                 150                 155                 160
   Thr Ile Asp Phe Ala Ala Lys Gln Gly His Gly Lys Ile Glu His Leu
                   165                 170                 175
   Lys Thr Pro Glu Gln Asn Val Glu Leu Ala Ala Ala Glu Leu Lys Ala
               180                 185                 190
   Asp Glu Lys Ser His Ala Val Ile Leu Gly Asp Thr Arg Tyr Gly Ser
           195                 200                 205
   Glu Glu Lys Gly Thr Tyr His Leu Ala Leu Phe Gly Asp Arg Ala Gln
       210                 215                 220
   Glu Ile Ala Gly Ser Ala Thr Val Lys Ile Gly Glu Lys Val His Glu
   225                 230                 235                 240
   Ile Gly Ile Ala Gly Lys Gln
                   245
<210> 3
   <211> 250
   <212> PRT
   <213> Neisseria meningitidis
<400> 3
   Val Ala Ala Asp Ile Gly Thr Gly Leu Ala Asp Ala Leu Thr Ala Pro
   1               5                   10                  15
   Leu Asp His Lys Asp Lys Gly Leu Lys Ser Leu Thr Leu Glu Asp Ser
               20                  25                  30
   Ile Pro Gln Asn Gly Thr Leu Thr Leu Ser Ala Gln Gly Ala Glu Lys
           35                  40                  45
   Thr Phe Lys Ala Gly Asp Lys Asp Asn Ser Leu Asn Thr Gly Lys Leu
       50                  55                  60
   Lys Asn Asp Lys Ile Ser Arg Phe Asp Phe Val Gln Lys Ile Glu Val
   65                  70                  75                  80
   Asp Gly Gln Thr Ile Thr Leu Ala Ser Gly Glu Phe Gln Ile Tyr Lys
                   85                  90                  95
   Gln Asn His Ser Ala Val Val Ala Leu Gln Ile Glu Lys Ile Asn Asn
               100                 105                 110
   Pro Asp Lys Thr Asp Ser Leu Ile Asn Gln Arg Ser Phe Leu Val Ser
           115                 120                 125
   Gly Leu Gly Gly Glu His Thr Ala Phe Asn Gln Leu Pro Gly Gly Lys
       130                 135                 140
   Ala Glu Tyr His Gly Lys Ala Phe Ser Ser Asp Asp Pro Asn Gly Arg
   145                 150                 155                 160
   Leu His Tyr Ser Ile Asp Phe Thr Lys Lys Gln Gly Tyr Gly Arg Ile
                   165                 170                 175
   Glu His Leu Lys Thr Leu Glu Gln Asn Val Glu Leu Ala Ala Ala Glu
               180                 185                 190
   Leu Lys Ala Asp Glu Lys Ser His Ala Val Ile Leu Gly Asp Thr Arg
           195                 200                 205
   Tyr Gly Ser Glu Glu Lys Gly Thr Tyr His Leu Ala Leu Phe Gly Asp
       210                 215                 220
   Arg Ala Gln Glu Ile Ala Gly Ser Ala Thr Val Lys Ile Gly Glu Lys
   225                 230                 235                 240
   Val His Glu Ile Gly Ile Ala Gly Lys Gln
                   245                 250
<210> 4
   <211> 644
   <212> PRT
   <213> Artificial Sequence
<220>
   <223> Hybrid meningococcal antigen
<400> 4
   Met Ala Ser Pro Asp Val Lys Ser Ala Asp Thr Leu Ser Lys Pro Ala
   1               5                   10                  15
   Ala Pro Val Val Ser Glu Lys Glu Thr Glu Ala Lys Glu Asp Ala Pro
               20                  25                  30
   Gln Ala Gly Ser Gln Gly Gln Gly Ala Pro Ser Ala Gln Gly Gly Gln
           35                  40                  45
   Asp Met Ala Ala Val Ser Glu Glu Asn Thr Gly Asn Gly Gly Ala Ala
       50                  55                  60
   Ala Thr Asp Lys Pro Lys Asn Glu Asp Glu Gly Ala Gln Asn Asp Met
   65                  70                  75                  80
   Pro Gln Asn Ala Ala Asp Thr Asp Ser Leu Thr Pro Asn His Thr Pro
                   85                  90                  95
   Ala Ser Asn Met Pro Ala Gly Asn Met Glu Asn Gln Ala Pro Asp Ala
               100                 105                 110
   Gly Glu Ser Glu Gln Pro Ala Asn Gln Pro Asp Met Ala Asn Thr Ala
           115                 120                 125
   Asp Gly Met Gln Gly Asp Asp Pro Ser Ala Gly Gly Glu Asn Ala Gly
       130                 135                 140
   Asn Thr Ala Ala Gln Gly Thr Asn Gln Ala Glu Asn Asn Gln Thr Ala
   145                 150                 155                 160
   Gly Ser Gln Asn Pro Ala Ser Ser Thr Asn Pro Ser Ala Thr Asn Ser
                   165                 170                 175
   Gly Gly Asp Phe Gly Arg Thr Asn Val Gly Asn Ser Val Val Ile Asp
               180                 185                 190
   Gly Pro Ser Gln Asn Ile Thr Leu Thr His Cys Lys Gly Asp Ser Cys
           195                 200                 205
   Ser Gly Asn Asn Phe Leu Asp Glu Glu Val Gln Leu Lys Ser Glu Phe
       210                 215                 220
   Glu Lys Leu Ser Asp Ala Asp Lys Ile Ser Asn Tyr Lys Lys Asp Gly
   225                 230                 235                 240
   Lys Asn Asp Gly Lys Asn Asp Lys Phe Val Gly Leu Val Ala Asp Ser
                   245                 250                 255
   Val Gln Met Lys Gly Ile Asn Gln Tyr Ile Ile Phe Tyr Lys Pro Lys
               260                 265                 270
   Pro Thr Ser Phe Ala Arg Phe Arg Arg Ser Ala Arg Ser Arg Arg Ser
           275                 280                 285
   Leu Pro Ala Glu Met Pro Leu Ile Pro Val Asn Gln Ala Asp Thr Leu
       290                 295                 300
   Ile Val Asp Gly Glu Ala Val Ser Leu Thr Gly His Ser Gly Asn Ile
   305                 310                 315                 320
   Phe Ala Pro Glu Gly Asn Tyr Arg Tyr Leu Thr Tyr Gly Ala Glu Lys
                   325                 330                 335
   Leu Pro Gly Gly Ser Tyr Ala Leu Arg Val Gln Gly Glu Pro Ser Lys
               340                 345                 350
   Gly Glu Met Leu Ala Gly Thr Ala Val Tyr Asn Gly Glu Val Leu His
           355                 360                 365
   Phe His Thr Glu Asn Gly Arg Pro Ser Pro Ser Arg Gly Arg Phe Ala
       370                 375                 380
   Ala Lys Val Asp Phe Gly Ser Lys Ser Val Asp Gly Ile Ile Asp Ser
   385                 390                 395                 400
   Gly Asp Gly Leu His Met Gly Thr Gln Lys Phe Lys Ala Ala Ile Asp
                   405                 410                 415
   Gly Asn Gly Phe Lys Gly Thr Trp Thr Glu Asn Gly Gly Gly Asp Val
               420                 425                 430
   Ser Gly Lys Phe Tyr Gly Pro Ala Gly Glu Glu Val Ala Gly Lys Tyr
           435                 440                 445
   Ser Tyr Arg Pro Thr Asp Ala Glu Lys Gly Gly Phe Gly Val Phe Ala
       450                 455                 460
   Gly Lys Lys Glu Gln Asp Gly Ser Gly Gly Gly Gly Ala Thr Tyr Lys
   465                 470                 475                 480
   Val Asp Glu Tyr His Ala Asn Ala Arg Phe Ala Ile Asp His Phe Asn
                   485                 490                 495
   Thr Ser Thr Asn Val Gly Gly Phe Tyr Gly Leu Thr Gly Ser Val Glu
               500                 505                 510
   Phe Asp Gln Ala Lys Arg Asp Gly Lys Ile Asp Ile Thr Ile Pro Val
           515                 520                 525
   Ala Asn Leu Gln Ser Gly Ser Gln His Phe Thr Asp His Leu Lys Ser
       530                 535                 540
   Ala Asp Ile Phe Asp Ala Ala Gln Tyr Pro Asp Ile Arg Phe Val Ser
   545                 550                 555                 560
   Thr Lys Phe Asn Phe Asn Gly Lys Lys Leu Val Ser Val Asp Gly Asn
                   565                 570                 575
   Leu Thr Met His Gly Lys Thr Ala Pro Val Lys Leu Lys Ala Glu Lys
               580                 585                 590
   Phe Asn Cys Tyr Gln Ser Pro Met Ala Lys Thr Glu Val Cys Gly Gly
           595                 600                 605
   Asp Phe Ser Thr Thr Ile Asp Arg Thr Lys Trp Gly Val Asp Tyr Leu
       610                 615                 620
   Val Asn Val Gly Met Thr Lys Ser Val Arg Ile Asp Ile Gln Ile Glu
   625                 630                 635                 640
   Ala Ala Lys Gln
<210> 5
   <211> 434
   <212> PRT
   <213> Artificial Sequence
<220>
   <223> Hybrid meningococcal antigen
<400> 5
   Met Val Ser Ala Val Ile Gly Ser Ala Ala Val Gly Ala Lys Ser Ala
   1               5                   10                  15
   Val Asp Arg Arg Thr Thr Gly Ala Gln Thr Asp Asp Asn Val Met Ala
               20                  25                  30
   Leu Arg Ile Glu Thr Thr Ala Arg Ser Tyr Leu Arg Gln Asn Asn Gln
           35                  40                  45
   Thr Lys Gly Tyr Thr Pro Gln Ile Ser Val Val Gly Tyr Asp Arg His
       50                  55                  60
   Leu Leu Leu Leu Gly Gln Val Ala Thr Glu Gly Glu Lys Gln Phe Val
   65                  70                  75                  80
   Gly Gln Ile Ala Arg Ser Glu Gln Ala Ala Glu Gly Val Tyr Asn Tyr
                   85                  90                  95
   Ile Thr Val Ala Ser Leu Pro Arg Thr Ala Gly Asp Ile Ala Gly Asp
               100                 105                 110
   Thr Trp Asn Thr Ser Lys Val Arg Ala Thr Leu Leu Gly Ile Ser Pro
           115                 120                 125
   Ala Thr Arg Ala Arg Val Lys Ile Val Thr Tyr Gly Asn Val Thr Tyr
       130                 135                 140
   Val Met Gly Ile Leu Thr Pro Glu Glu Gln Ala Gln Ile Thr Gln Lys
   145                 150                 155                 160
   Val Ser Thr Thr Val Gly Val Gln Lys Val Ile Thr Leu Tyr Gln Asn
                   165                 170                 175
   Tyr Val Gln Arg Gly Ser Gly Gly Gly Gly Val Ala Ala Asp Ile Gly
               180                 185                 190
   Ala Gly Leu Ala Asp Ala Leu Thr Ala Pro Leu Asp His Lys Asp Lys
           195                 200                 205
   Gly Leu Gln Ser Leu Thr Leu Asp Gln Ser Val Arg Lys Asn Glu Lys
       210                 215                 220
   Leu Lys Leu Ala Ala Gln Gly Ala Glu Lys Thr Tyr Gly Asn Gly Asp
   225                 230                 235                 240
   Ser Leu Asn Thr Gly Lys Leu Lys Asn Asp Lys Val Ser Arg Phe Asp
                   245                 250                 255
   Phe Ile Arg Gln Ile Glu Val Asp Gly Gln Leu Ile Thr Leu Glu Ser
               260                 265                 270
   Gly Glu Phe Gln Val Tyr Lys Gln Ser His Ser Ala Leu Thr Ala Phe
           275                 280                 285
   Gln Thr Glu Gln Ile Gln Asp Ser Glu His Ser Gly Lys Met Val Ala
       290                 295                 300
   Lys Arg Gln Phe Arg Ile Gly Asp Ile Ala Gly Glu His Thr Ser Phe
   305                 310                 315                 320
   Asp Lys Leu Pro Glu Gly Gly Arg Ala Thr Tyr Arg Gly Thr Ala Phe
                   325                 330                 335
   Gly Ser Asp Asp Ala Gly Gly Lys Leu Thr Tyr Thr Ile Asp Phe Ala
               340                 345                 350
   Ala Lys Gln Gly Asn Gly Lys Ile Glu His Leu Lys Ser Pro Glu Leu
           355                 360                 365
   Asn Val Asp Leu Ala Ala Ala Asp Ile Lys Pro Asp Gly Lys Arg His
       370                 375                 380
   Ala Val Ile Ser Gly Ser Val Leu Tyr Asn Gln Ala Glu Lys Gly Ser
   385                 390                 395                 400
   Tyr Ser Leu Gly Ile Phe Gly Gly Lys Ala Gln Glu Val Ala Gly Ser
                   405                 410                 415
   Ala Glu Val Lys Thr Val Asn Gly Ile Arg His Ile Gly Leu Ala Ala
               420                 425                 430
   Lys Gln
<210> 6
   <211> 327
   <212> PRT
   <213> Neisseria meningitidis
<400> 6
   Ala Thr Asn Asp Asp Asp Val Lys Lys Ala Ala Thr Val Ala Ile Ala
   1               5                   10                  15
   Ala Ala Tyr Asn Asn Gly Gln Glu Ile Asn Gly Phe Lys Ala Gly Glu
               20                  25                  30
   Thr Ile Tyr Asp Ile Asp Glu Asp Gly Thr Ile Thr Lys Lys Asp Ala
           35                  40                  45
   Thr Ala Ala Asp Val Glu Ala Asp Asp Phe Lys Gly Leu Gly Leu Lys
       50                  55                  60
   Lys Val Val Thr Asn Leu Thr Lys Thr Val Asn Glu Asn Lys Gln Asn
   65                  70                  75                  80
   Val Asp Ala Lys Val Lys Ala Ala Glu Ser Glu Ile Glu Lys Leu Thr
                   85                  90                  95
   Thr Lys Leu Ala Asp Thr Asp Ala Ala Leu Ala Asp Thr Asp Ala Ala
               100                 105                 110
   Leu Asp Ala Thr Thr Asn Ala Leu Asn Lys Leu Gly Glu Asn Ile Thr
           115                 120                 125
   Thr Phe Ala Glu Glu Thr Lys Thr Asn Ile Val Lys Ile Asp Glu Lys
       130                 135                 140
   Leu Glu Ala Val Ala Asp Thr Val Asp Lys His Ala Glu Ala Phe Asn
   145                 150                 155                 160
   Asp Ile Ala Asp Ser Leu Asp Glu Thr Asn Thr Lys Ala Asp Glu Ala
                   165                 170                 175
   Val Lys Thr Ala Asn Glu Ala Lys Gln Thr Ala Glu Glu Thr Lys Gln
               180                 185                 190
   Asn Val Asp Ala Lys Val Lys Ala Ala Glu Thr Ala Ala Gly Lys Ala
           195                 200                 205
   Glu Ala Ala Ala Gly Thr Ala Asn Thr Ala Ala Asp Lys Ala Glu Ala
       210                 215                 220
   Val Ala Ala Lys Val Thr Asp Ile Lys Ala Asp Ile Ala Thr Asn Lys
   225                 230                 235                 240
   Asp Asn Ile Ala Lys Lys Ala Asn Ser Ala Asp Val Tyr Thr Arg Glu
                   245                 250                 255
   Glu Ser Asp Ser Lys Phe Val Arg Ile Asp Gly Leu Asn Ala Thr Thr
               260                 265                 270
   Glu Lys Leu Asp Thr Arg Leu Ala Ser Ala Glu Lys Ser Ile Ala Asp
           275                 280                 285
   His Asp Thr Arg Leu Asn Gly Leu Asp Lys Thr Val Ser Asp Leu Arg
       290                 295                 300
   Lys Glu Thr Arg Gln Gly Leu Ala Glu Gln Ala Ala Leu Ser Gly Leu
   305                 310                 315                 320
   Phe Gln Pro Tyr Asn Val Gly
                   325
<210> 7
   <211> 792
   <212> PRT
   <213> Neisseria meningitidis
<400> 7
   Met Lys Pro Leu Gln Met Leu Pro Ile Ala Ala Leu Val Gly Ser Ile
   1               5                   10                  15
   Phe Gly Asn Pro Val Leu Ala Ala Asp Glu Ala Ala Thr Glu Thr Thr
               20                  25                  30
   Pro Val Lys Ala Glu Ile Lys Ala Val Arg Val Lys Gly Gln Arg Asn
           35                  40                  45
   Ala Pro Ala Ala Val Glu Arg Val Asn Leu Asn Arg Ile Lys Gln Glu
       50                  55                  60
   Met Ile Arg Asp Asn Lys Asp Leu Val Arg Tyr Ser Thr Asp Val Gly
   65                  70                  75                  80
   Leu Ser Asp Ser Gly Arg His Gln Lys Gly Phe Ala Val Arg Gly Val
                   85                  90                  95
   Glu Gly Asn Arg Val Gly Val Ser Ile Asp Gly Val Asn Leu Pro Asp
               100                 105                 110
   Ser Glu Glu Asn Ser Leu Tyr Ala Arg Tyr Gly Asn Phe Asn Ser Ser
           115                 120                 125
   Arg Leu Ser Ile Asp Pro Glu Leu Val Arg Asn Ile Glu Ile Val Lys
       130                 135                 140
   Gly Ala Asp Ser Phe Asn Thr Gly Ser Gly Ala Leu Gly Gly Gly Val
   145                 150                 155                 160
   Asn Tyr Gln Thr Leu Gln Gly Arg Asp Leu Leu Leu Asp Asp Arg Gln
                   165                 170                 175
   Phe Gly Val Met Met Lys Asn Gly Tyr Ser Thr Arg Asn Arg Glu Trp
               180                 185                 190
   Thr Asn Thr Leu Gly Phe Gly Val Ser Asn Asp Arg Val Asp Ala Ala
           195                 200                 205
   Leu Leu Tyr Ser Gln Arg Arg Gly His Glu Thr Glu Ser Ala Gly Asn
       210                 215                 220
   Arg Gly Tyr Ala Val Glu Gly Glu Gly Ser Gly Ala Asn Ile Arg Gly
   225                 230                 235                 240
   Ser Ala Arg Gly Ile Pro Asp Ser Ser Lys His Lys Tyr Asn His His
                   245                 250                 255
   Ala Leu Gly Lys Ile Ala Tyr Gln Ile Asn Asp Asn His Arg Ile Gly
               260                 265                 270
   Ala Ser Leu Asn Gly Gln Gln Gly His Asn Tyr Thr Val Glu Glu Ser
           275                 280                 285
   Tyr Asn Leu Thr Ala Ser Ser Trp Arg Glu Ala Asp Asp Val Asn Arg
       290                 295                 300
   Arg Arg Asn Ala Asn Leu Phe Tyr Glu Trp Met Pro Asp Ser Asn Trp
   305                 310                 315                 320
   Leu Ser Ser Leu Lys Ala Asp Phe Asp Tyr Gln Lys Thr Lys Val Ala
                   325                 330                 335
   Ala Val Asn Asn Lys Gly Ser Phe Pro Met Asp Tyr Ser Thr Trp Thr
               340                 345                 350
   Arg Asn Tyr Asn Gln Lys Asp Leu Asp Glu Ile Tyr Asn Arg Ser Met
           355                 360                 365
   Asp Thr Arg Phe Lys Arg Phe Thr Leu Arg Leu Asp Ser His Pro Leu
       370                 375                 380
   Gln Leu Gly Gly Gly Arg His Arg Leu Ser Phe Lys Thr Phe Val Ser
   385                 390                 395                 400
   Arg Arg Asp Phe Glu Asn Leu Asn Arg Asp Asp Tyr Tyr Phe Ser Gly
                   405                 410                 415
   Arg Val Val Arg Thr Thr Ser Ser Ile Gln His Pro Val Lys Thr Thr
               420                 425                 430
   Asn Tyr Gly Phe Ser Leu Ser Asp Gln Ile Gln Trp Asn Asp Val Phe
           435                 440                 445
   Ser Ser Arg Ala Gly Ile Arg Tyr Asp His Thr Lys Met Thr Pro Gln
       450                 455                 460
   Glu Leu Asn Ala Glu Cys His Ala Cys Asp Lys Thr Pro Pro Ala Ala
   465                 470                 475                 480
   Asn Thr Tyr Lys Gly Trp Ser Gly Phe Val Gly Leu Ala Ala Gln Leu
                   485                 490                 495
   Asn Gln Ala Trp Arg Val Gly Tyr Asp Ile Thr Ser Gly Tyr Arg Val
               500                 505                 510
   Pro Asn Ala Ser Glu Val Tyr Phe Thr Tyr Asn His Gly Ser Gly Asn
           515                 520                 525
   Trp Leu Pro Asn Pro Asn Leu Lys Ala Glu Arg Ser Thr Thr His Thr
       530                 535                 540
   Leu Ser Leu Gln Gly Arg Ser Glu Lys Gly Met Leu Asp Ala Asn Leu
   545                 550                 555                 560
   Tyr Gln Ser Asn Tyr Arg Asn Phe Leu Ser Glu Glu Gln Lys Leu Thr
                   565                 570                 575
   Thr Ser Gly Thr Pro Gly Cys Thr Glu Glu Asn Ala Tyr Tyr Gly Ile
               580                 585                 590
   Cys Ser Asp Pro Tyr Lys Glu Lys Leu Asp Trp Gln Met Lys Asn Ile
           595                 600                 605
   Asp Lys Ala Arg Ile Arg Gly Ile Glu Leu Thr Gly Arg Leu Asn Val
       610                 615                 620
   Asp Lys Val Ala Ser Phe Val Pro Glu Gly Trp Lys Leu Phe Gly Ser
   625                 630                 635                 640
   Leu Gly Tyr Ala Lys Ser Lys Leu Ser Gly Asp Asn Ser Leu Leu Ser
                   645                 650                 655
   Thr Gln Pro Leu Lys Val Ile Ala Gly Ile Asp Tyr Glu Ser Pro Ser
               660                 665                 670
   Glu Lys Trp Gly Val Phe Ser Arg Leu Thr Tyr Leu Gly Ala Lys Lys
           675                 680                 685
   Val Lys Asp Ala Gln Tyr Thr Val Tyr Glu Asn Lys Gly Trp Gly Thr
       690                 695                 700
   Pro Leu Gln Lys Lys Val Lys Asp Tyr Pro Trp Leu Asn Lys Ser Ala
   705                 710                 715                 720
   Tyr Val Phe Asp Met Tyr Gly Phe Tyr Lys Pro Ala Lys Asn Leu Thr
                   725                 730                 735
   Leu Arg Ala Gly Val Tyr Asn Leu Phe Asn Arg Lys Tyr Thr Thr Trp
               740                 745                 750
   Asp Ser Leu Arg Gly Leu Tyr Ser Tyr Ser Thr Thr Asn Ala Val Asp
           755                 760                 765
   Arg Asp Gly Lys Gly Leu Asp Arg Tyr Arg Ala Pro Gly Arg Asn Tyr
       770                 775                 780
   Ala Val Ser Leu Glu Trp Lys Phe
   785                 790
<210> 8
   <211> 793
   <212> PRT
   <213> Neisseria meningitidis
<400> 8
   Met Lys Pro Leu Gln Met Leu Pro Ile Ala Ala Leu Val Gly Ser Ile
   1               5                   10                  15
   Phe Gly Asn Pro Val Phe Ala Ala Asp Glu Ala Ala Thr Glu Thr Thr
               20                  25                  30
   Pro Val Lys Ala Glu Val Lys Ala Val Arg Val Lys Gly Gln Arg Asn
           35                  40                  45
   Ala Pro Ala Ala Val Glu Arg Val Asn Leu Asn Arg Ile Lys Gln Glu
       50                  55                  60
   Met Ile Arg Asp Asn Lys Asp Leu Val Arg Tyr Ser Thr Asp Val Gly
   65                  70                  75                  80
   Leu Ser Asp Ser Gly Arg His Gln Lys Gly Phe Ala Val Arg Gly Val
                   85                  90                  95
   Glu Gly Asn Arg Val Gly Val Ser Ile Asp Gly Val Asn Leu Pro Asp
               100                 105                 110
   Ser Glu Glu Asn Ser Leu Tyr Ala Arg Tyr Gly Asn Phe Asn Ser Ser
           115                 120                 125
   Arg Leu Ser Ile Asp Pro Glu Leu Val Arg Asn Ile Asp Ile Val Lys
       130                 135                 140
   Gly Ala Asp Ser Phe Asn Thr Gly Ser Gly Ala Leu Gly Gly Gly Val
   145                 150                 155                 160
   Asn Tyr Gln Thr Leu Gln Gly Arg Asp Leu Leu Leu Pro Glu Arg Gln
                   165                 170                 175
   Phe Gly Val Met Met Lys Asn Gly Tyr Ser Thr Arg Asn Arg Glu Trp
               180                 185                 190
   Thr Asn Thr Leu Gly Phe Gly Val Ser Asn Asp Arg Val Asp Ala Ala
           195                 200                 205
   Leu Leu Tyr Ser Gln Arg Arg Gly His Glu Thr Glu Ser Ala Gly Lys
       210                 215                 220
   Arg Gly Tyr Pro Val Glu Gly Ala Gly Ser Gly Ala Asn Ile Arg Gly
   225                 230                 235                 240
   Ser Ala Arg Gly Ile Pro Asp Pro Ser Gln His Lys Tyr Asn His His
                   245                 250                 255
   Ala Leu Gly Lys Ile Ala Tyr Gln Ile Asn Asp Asn His Arg Ile Gly
               260                 265                 270
   Ala Ser Leu Asn Gly Gln Gln Gly His Asn Tyr Thr Val Glu Glu Ser
           275                 280                 285
   Tyr Asn Leu Leu Ala Ser Tyr Trp Arg Glu Ala Asp Asp Val Asn Arg
       290                 295                 300
   Arg Arg Asn Thr Asn Leu Phe Tyr Glu Trp Thr Pro Glu Ser Asp Arg
   305 310 315 320
   Leu Ser Met Val Lys Ala Asp Val Asp Tyr Gln Lys Thr Lys Val Ser
   325 330 335
   Ala Val Asn Tyr Lys Gly Ser Phe Pro Ile Glu Asp Ser Ser Thr Leu
   340 345 350
   Thr Arg Asn Tyr Asn Gln Lys Asp Leu Asp Glu Ile Tyr Asn Arg Ser
   355 360 365
   Met Asp Thr Arg Phe Lys Arg Ile Thr Leu Arg Leu Asp Ser His Pro
   370 375 380
   Leu Gln Leu Gly Gly Gly Arg His Arg Leu Ser Phe Lys Thr Phe Ala
   385 390 395 400
   Ser Arg Arg Asp Phe Glu Asn Leu Asn Arg Asp Asp Tyr Tyr Phe Ser
   405 410 415
   Gly Arg Val Val Arg Thr Thr Ser Ser Ile Gln His Pro Val Lys Thr
   420 425 430
   Thr Asn Tyr Gly Phe Ser Leu Ser Asp Gln Ile Gln Trp Asn Asp Val
   435 440 445
   Phe Ser Ser Arg Ala Gly Ile Arg Tyr Asp His Thr Lys Met Thr Pro
   450 455 460
   Gln Glu Leu Asn Ala Glu Cys His Ala Cys Asp Lys Thr Pro Pro Ala
   465 470 475 480
   Ala Asn Thr Tyr Lys Gly Trp Ser Gly Phe Val Gly Leu Ala Ala Gln
   485 490 495
   Leu Asn Gln Ala Trp Arg Val Gly Tyr Asp Ile Thr Ser Gly Tyr Arg
   500 505 510
   Val Pro Asn Ala Ser Glu Val Tyr Phe Thr Tyr Asn His Gly Ser Gly
   515 520 525
   Asn Trp Leu Pro Asn Pro Asn Leu Lys Ala Glu Arg Thr Thr Thr His
   530 535 540
   Thr Leu Ser Leu Gln Gly Arg Ser Glu Lys Gly Thr Leu Asp Ala Asn
   545 550 555 560
   Leu Tyr Gln Ser Asn Tyr Arg Asn Phe Leu Ser Glu Glu Gln Lys Leu
   565 570 575
   Thr Thr Ser Gly Asp Val Ser Cys Thr Gln Met Asn Tyr Tyr Tyr Gly
   580 585 590
   Met Cys Ser Asn Pro Tyr Ser Glu Lys Leu Glu Trp Gln Met Gln Asn
   595 600 605
   Ile Asp Lys Ala Arg Ile Arg Gly Ile Glu Leu Thr Gly Arg Leu Asn
   610 615 620
   Val Asp Lys Val Ala Ser Phe Val Pro Glu Gly Trp Lys Leu Phe Gly
   625 630 635 640
   Ser Leu Gly Tyr Ala Lys Ser Lys Leu Ser Gly Asp Asn Ser Leu Leu
                   645                 650                 655
   Ser Thr Gln Pro Leu Lys Val Ile Ala Gly Ile Asp Tyr Glu Ser Pro
               660                 665                 670
   Ser Glu Lys Trp Gly Val Phe Ser Arg Leu Thr Tyr Leu Gly Ala Lys
           675                 680                 685
   Lys Val Lys Asp Ala Gln Tyr Thr Val Tyr Glu Asn Lys Gly Trp Gly
       690                 695                 700
   Thr Pro Leu Gln Lys Lys Val Lys Asp Tyr Pro Trp Leu Asn Lys Ser
   705                 710                 715                 720
   Ala Tyr Val Phe Asp Met Tyr Gly Phe Tyr Lys Pro Val Lys Asn Leu
                   725                 730                 735
   Thr Leu Arg Ala Gly Val Tyr Asn Val Phe Asn Arg Lys Tyr Thr Thr
               740                 745                 750
   Trp Asp Ser Leu Arg Gly Leu Tyr Ser Tyr Ser Thr Thr Asn Ser Val
           755                 760                 765
   Asp Arg Asp Gly Lys Gly Leu Asp Arg Tyr Arg Ala Pro Ser Arg Asn
       770                 775                 780
   Tyr Ala Val Ser Leu Glu Trp Lys Phe
   785                 790
<210> 9
   <211> 488
   <212> PRT
   <213> Neisseria meningitidis
<400> 9
   Met Phe Lys Arg Ser Val Ile Ala Met Ala Cys Ile Phe Ala Leu Ser
   1               5                   10                  15
   Ala Cys Gly Gly Gly Gly Gly Gly Ser Pro Asp Val Lys Ser Ala Asp
               20                  25                  30
   Thr Leu Ser Lys Pro Ala Ala Pro Val Val Ser Glu Lys Glu Thr Glu
           35                  40                  45
   Ala Lys Glu Asp Ala Pro Gln Ala Gly Ser Gln Gly Gln Gly Ala Pro
       50                  55                  60
   Ser Ala Gln Gly Ser Gln Asp Met Ala Ala Val Ser Glu Glu Asn Thr
   65                  70                  75                  80
   Gly Asn Gly Gly Ala Val Thr Ala Asp Asn Pro Lys Asn Glu Asp Glu
                   85                  90                  95
   Val Ala Gln Asn Asp Met Pro Gln Asn Ala Ala Gly Thr Asp Ser Ser
               100                 105                 110
   Thr Pro Asn His Thr Pro Asp Pro Asn Met Leu Ala Gly Asn Met Glu
           115                 120                 125
   Asn Gln Ala Thr Asp Ala Gly Glu Ser Ser Gln Pro Ala Asn Gln Pro
       130                 135                 140
   Asp Met Ala Asn Ala Ala Asp Gly Met Gln Gly Asp Asp Pro Ser Ala
   145                 150                 155                 160
   Gly Gly Gln Asn Ala Gly Asn Thr Ala Ala Gln Gly Ala Asn Gln Ala
                   165                 170                 175
   Gly Asn Asn Gln Ala Ala Gly Ser Ser Asp Pro Ile Pro Ala Ser Asn
               180                 185                 190
   Pro Ala Pro Ala Asn Gly Gly Ser Asn Phe Gly Arg Val Asp Leu Ala
           195                 200                 205
   Asn Gly Val Leu Ile Asp Gly Pro Ser Gln Asn Ile Thr Leu Thr His
       210                 215                 220
   Cys Lys Gly Asp Ser Cys Ser Gly Asn Asn Phe Leu Asp Glu Glu Val
   225                 230                 235                 240
   Gln Leu Lys Ser Glu Phe Glu Lys Leu Ser Asp Ala Asp Lys Ile Ser
                   245                 250                 255
   Asn Tyr Lys Lys Asp Gly Lys Asn Asp Lys Phe Val Gly Leu Val Ala
               260                 265                 270
   Asp Ser Val Gln Met Lys Gly Ile Asn Gln Tyr Ile Ile Phe Tyr Lys
           275                 280                 285
   Pro Lys Pro Thr Ser Phe Ala Arg Phe Arg Arg Ser Ala Arg Ser Arg
       290                 295                 300
   Arg Ser Leu Pro Ala Glu Met Pro Leu Ile Pro Val Asn Gln Ala Asp
   305                 310                 315                 320
   Thr Leu Ile Val Asp Gly Glu Ala Val Ser Leu Thr Gly His Ser Gly
                   325                 330                 335
   Asn Ile Phe Ala Pro Glu Gly Asn Tyr Arg Tyr Leu Thr Tyr Gly Ala
               340                 345                 350
   Glu Lys Leu Pro Gly Gly Ser Tyr Ala Leu Arg Val Gln Gly Glu Pro
           355                 360                 365
   Ala Lys Gly Glu Met Leu Ala Gly Ala Ala Val Tyr Asn Gly Glu Val
       370                 375                 380
   Leu His Phe His Thr Glu Asn Gly Arg Pro Tyr Pro Thr Arg Gly Arg
   385                 390                 395                 400
   Phe Ala Ala Lys Val Asp Phe Gly Ser Lys Ser Val Asp Gly Ile Ile
                   405                 410                 415
   Asp Ser Gly Asp Asp Leu His Met Gly Thr Gln Lys Phe Lys Ala Ala
               420                 425                 430
   Ile Asp Gly Asn Gly Phe Lys Gly Thr Trp Thr Glu Asn Gly Ser Gly
           435                 440                 445
   Asp Val Ser Gly Lys Phe Tyr Gly Pro Ala Gly Glu Glu Val Ala Gly
       450                 455                 460
   Lys Tyr Ser Tyr Arg Pro Thr Asp Ala Glu Lys Gly Gly Phe Gly Val
   465                 470                 475                 480
   Phe Ala Gly Lys Lys Glu Gln Asp
   485
<210> 10
   <211> 364
   <212> PRT
   <213> Neisseria meningitidis
<400> 10
   Met Ser Met Lys His Phe Pro Ser Lys Val Leu Thr Thr Ala Ile Leu
   1               5                   10                  15
   Ala Thr Phe Cys Ser Gly Ala Leu Ala Ala Thr Ser Asp Asp Asp Val
               20                  25                  30
   Lys Lys Ala Ala Thr Val Ala Ile Val Ala Ala Tyr Asn Asn Gly Gln
           35                  40                  45
   Glu Ile Asn Gly Phe Lys Ala Gly Glu Thr Ile Tyr Asp Ile Gly Glu
       50                  55                  60
   Asp Gly Thr Ile Thr Gln Lys Asp Ala Thr Ala Ala Asp Val Glu Ala
   65                  70                  75                  80
   Asp Asp Phe Lys Gly Leu Gly Leu Lys Lys Val Val Thr Asn Leu Thr
                   85                  90                  95
   Lys Thr Val Asn Glu Asn Lys Gln Asn Val Asp Ala Lys Val Lys Ala
               100                 105                 110
   Ala Glu Ser Glu Ile Glu Lys Leu Thr Thr Lys Leu Ala Asp Thr Asp
           115                 120                 125
   Ala Ala Leu Ala Asp Thr Asp Ala Ala Leu Asp Glu Thr Thr Asn Ala
       130                 135                 140
   Leu Asn Lys Leu Gly Glu Asn Ile Thr Thr Phe Ala Glu Glu Thr Lys
   145                 150                 155                 160
   Thr Asn Ile Val Lys Ile Asp Glu Lys Leu Glu Ala Val Ala Asp Thr
                   165                 170                 175
   Val Asp Lys His Ala Glu Ala Phe Asn Asp Ile Ala Asp Ser Leu Asp
               180                 185                 190
   Glu Thr Asn Thr Lys Ala Asp Glu Ala Val Lys Thr Ala Asn Glu Ala
           195                 200                 205
   Lys Gln Thr Ala Glu Glu Thr Lys Gln Asn Val Asp Ala Lys Val Lys
       210                 215                 220
   Ala Ala Glu Thr Ala Ala Gly Lys Ala Glu Ala Ala Ala Gly Thr Ala
   225                 230                 235                 240
   Asn Thr Ala Ala Asp Lys Ala Glu Ala Val Ala Ala Lys Val Thr Asp
                   245                 250                 255
   Ile Lys Ala Asp Ile Ala Thr Asn Lys Ala Asp Ile Ala Lys Asn Ser
               260                 265                 270
   Ala Arg Ile Asp Ser Leu Asp Lys Asn Val Ala Asn Leu Arg Lys Glu
           275                 280                 285
   Thr Arg Gln Gly Leu Ala Glu Gln Ala Ala Leu Ser Gly Leu Phe Gln
   290 295 300
   Pro Tyr Asn Val Gly Arg Phe Asn Val Thr Ala Ala Val Gly Gly Tyr
   305 310 315 320
   Lys Ser Glu Ser Ala Val Ala Ile Gly Thr Gly Phe Arg Phe Thr Glu
   325 330 335
   Asn Phe Ala Ala Lys Ala Gly Val Ala Val Gly Thr Ser Ser Gly Ser
   340 345 350
   Ser Ala Ala Tyr His Val Gly Val Asn Tyr Glu Trp
   355 360
<210> 11
   <211> 174
   <212> PRT
   <213> Neisseria meningitidis
<400> 11
   Met Lys Lys Ala Leu Ala Thr Leu Ile Ala Leu Ala Leu Pro Ala Ala
   1 5 10 15
   Ala Leu Ala Glu Gly Ala Ser Gly Phe Tyr Val Gln Ala Asp Ala Ala
   20 25 30
   His Ala Lys Ala Ser Ser Ser Leu Gly Ser Ala Lys Gly Phe Ser Pro
   35 40 45
   Arg Ile Ser Ala Gly Tyr Arg Ile Asn Asp Leu Arg Phe Ala Val Asp
   50 55 60
   Tyr Thr Arg Tyr Lys Asn Tyr Lys Ala Pro Ser Thr Asp Phe Lys Leu
   65 70 75 80
   Tyr Ser Ile Gly Ala Ser Ala Ile Tyr Asp Phe Asp Thr Gln Ser Pro
   85 90 95
   Val Lys Pro Tyr Leu Gly Ala Arg Leu Ser Leu Asn Arg Ala Ser Val
   100 105 110
   Asp Leu Gly Gly Ser Asp Ser Phe Ser Gln Thr Ser Ile Gly Leu Gly
   115 120 125
   Val Leu Thr Gly Val Ser Tyr Ala Val Thr Pro Asn Val Asp Leu Asp
   130 135 140
   Ala Gly Tyr Arg Tyr Asn Tyr Ile Gly Lys Val Asn Thr Val Lys Asn
   145 150 155 160
   Val Arg Ser Gly Glu Leu Ser Ala Gly Val Arg Val Lys Phe
   165 170
<210> 12
   <211> 591
   <212> PRT
   <213> Neisseria meningitidis
<400> 12
   Met Asn Lys Ile Tyr Arg Ile Ile Trp Asn Ser Ala Leu Asn Ala Trp
   1               5                   10                  15
   Val Val Val Ser Glu Leu Thr Arg Asn His Thr Lys Arg Ala Ser Ala
               20                  25                  30
   Thr Val Lys Thr Ala Val Leu Ala Thr Leu Leu Phe Ala Thr Val Gln
           35                  40                  45
   Ala Ser Ala Asn Asn Glu Glu Gln Glu Glu Asp Leu Tyr Leu Asp Pro
       50                  55                  60
   Val Gln Arg Thr Val Ala Val Leu Ile Val Asn Ser Asp Lys Glu Gly
   65                  70                  75                  80
   Thr Gly Glu Lys Glu Lys Val Glu Glu Asn Ser Asp Trp Ala Val Tyr
                   85                  90                  95
   Phe Asn Glu Lys Gly Val Leu Thr Ala Arg Glu Ile Thr Leu Lys Ala
               100                 105                 110
   Gly Asp Asn Leu Lys Ile Lys Gln Asn Gly Thr Asn Phe Thr Tyr Ser
           115                 120                 125
   Leu Lys Lys Asp Leu Thr Asp Leu Thr Ser Val Gly Thr Glu Lys Leu
       130                 135                 140
   Ser Phe Ser Ala Asn Gly Asn Lys Val Asn Ile Thr Ser Asp Thr Lys
   145                 150                 155                 160
   Gly Leu Asn Phe Ala Lys Glu Thr Ala Gly Thr Asn Gly Asp Thr Thr
                   165                 170                 175
   Val His Leu Asn Gly Ile Gly Ser Thr Leu Thr Asp Thr Leu Leu Asn
               180                 185                 190
   Thr Gly Ala Thr Thr Asn Val Thr Asn Asp Asn Val Thr Asp Asp Glu
           195                 200                 205
   Lys Lys Arg Ala Ala Ser Val Lys Asp Val Leu Asn Ala Gly Trp Asn
       210                 215                 220
   Ile Lys Gly Val Lys Pro Gly Thr Thr Ala Ser Asp Asn Val Asp Phe
   225                 230                 235                 240
   Val Arg Thr Tyr Asp Thr Val Glu Phe Leu Ser Ala Asp Thr Lys Thr
                   245                 250                 255
   Thr Thr Val Asn Val Glu Ser Lys Asp Asn Gly Lys Lys Thr Glu Val
               260                 265                 270
   Lys Ile Gly Ala Lys Thr Ser Val Ile Lys Glu Lys Asp Gly Lys Leu
           275                 280                 285
   Val Thr Gly Lys Asp Lys Gly Glu Asn Gly Ser Ser Thr Asp Glu Gly
       290                 295                 300
   Glu Gly Leu Val Thr Ala Lys Glu Val Ile Asp Ala Val Asn Lys Ala
   305                 310                 315                 320
   Gly Trp Arg Met Lys Thr Thr Thr Ala Asn Gly Gln Thr Gly Gln Ala
                   325                 330                 335
   Asp Lys Phe Glu Thr Val Thr Ser Gly Thr Asn Val Thr Phe Ala Ser
               340                 345                 350
   Gly Lys Gly Thr Thr Ala Thr Val Ser Lys Asp Asp Gln Gly Asn Ile
   355 360 365
   Thr Val Met Tyr Asp Val Asn Val Gly Asp Ala Leu Asn Val Asn Gln
   370 375 380
   Leu Gln Asn Ser Gly Trp Asn Leu Asp Ser Lys Ala Val Ala Gly Ser
   385 390 395 400
   Ser Gly Lys Val Ile Ser Gly Asn Val Ser Pro Ser Lys Gly Lys Met
   405 410 415
   Asp Glu Thr Val Asn Ile Asn Ala Gly Asn Asn Ile Glu Ile Thr Arg
   420 425 430
   Asn Gly Lys Asn Ile Asp Ile Ala Thr Ser Met Thr Pro Gln Phe Ser
   435 440 445
   Ser Val Ser Leu Gly Ala Gly Ala Asp Ala Pro Thr Leu Ser Val Asp
   450 455 460
   Gly Asp Ala Leu Asn Val Gly Ser Lys Lys Asp Asn Lys Pro Val Arg
   465 470 475 480
   Ile Thr Asn Val Ala Pro Gly Val Lys Glu Gly Asp Val Thr Asn Val
   485 490 495
   Ala Gln Leu Lys Gly Val Ala Gln Asn Leu Asn Asn Arg Ile Asp Asn
   500 505 510
   Val Asp Gly Asn Ala Arg Ala Gly Ile Ala Gln Ala Ile Ala Thr Ala
   515 520 525
   Gly Leu Val Gln Ala Tyr Leu Pro Gly Lys Ser Met Met Ala Ile Gly
   530 535 540
   Gly Gly Thr Tyr Arg Gly Glu Ala Gly Tyr Ala Ile Gly Tyr Ser Ser
   545 550 555 560
   Ile Ser Asp Gly Gly Asn Trp Ile Ile Lys Gly Thr Ala Ser Gly Asn
   565 570 575
   Ser Arg Gly His Phe Gly Ala Ser Ala Ser Val Gly Tyr Gln Trp
   580 585 590
<210> 13
   <211> 1457
   <212> PRT
   <213> Neisseria meningitidis
<400> 13
   Met Lys Thr Thr Asp Lys Arg Thr Thr Glu Thr His Arg Lys Ala Pro
   1               5                   10                  15
   Lys Thr Gly Arg Ile Arg Phe Ser Pro Ala Tyr Leu Ala Ile Cys Leu
               20                  25                  30
   Ser Phe Gly Ile Leu Pro Gln Ala Trp Ala Gly His Thr Tyr Phe Gly
           35                  40                  45
   Ile Asn Tyr Gln Tyr Tyr Arg Asp Phe Ala Glu Asn Lys Gly Lys Phe
       50                  55                  60
   Ala Val Gly Ala Lys Asp Ile Glu Val Tyr Asn Lys Lys Gly Glu Leu
   65                  70                  75                  80
   Val Gly Lys Ser Met Thr Lys Ala Pro Met Ile Asp Phe Ser Val Val
                   85                  90                  95
   Ser Arg Asn Gly Val Ala Ala Leu Val Gly Asp Gln Tyr Ile Val Ser
               100                 105                 110
   Val Ala His Asn Gly Gly Tyr Asn Asn Val Asp Phe Gly Ala Glu Gly
           115                 120                 125
   Arg Asn Pro Asp Gln His Arg Phe Thr Tyr Lys Ile Val Lys Arg Asn
       130                 135                 140
   Asn Tyr Lys Ala Gly Thr Lys Gly His Pro Tyr Gly Gly Asp Tyr His
   145                 150                 155                 160
   Met Pro Arg Leu His Lys Phe Val Thr Asp Ala Glu Pro Val Glu Met
                   165                 170                 175
   Thr Ser Tyr Met Asp Gly Arg Lys Tyr Ile Asp Gln Asn Asn Tyr Pro
               180                 185                 190
   Asp Arg Val Arg Ile Gly Ala Gly Arg Gln Tyr Trp Arg Ser Asp Glu
           195                 200                 205
   Asp Glu Pro Asn Asn Arg Glu Ser Ser Tyr His Ile Ala Ser Ala Tyr
       210                 215                 220
   Ser Trp Leu Val Gly Gly Asn Thr Phe Ala Gln Asn Gly Ser Gly Gly
   225                 230                 235                 240
   Gly Thr Val Asn Leu Gly Ser Glu Lys Ile Lys His Ser Pro Tyr Gly
                   245                 250                 255
   Phe Leu Pro Thr Gly Gly Ser Phe Gly Asp Ser Gly Ser Pro Met Phe
               260                 265                 270
   Ile Tyr Asp Ala Gln Lys Gln Lys Trp Leu Ile Asn Gly Val Leu Gln
           275                 280                 285
   Thr Gly Asn Pro Tyr Ile Gly Lys Ser Asn Gly Phe Gln Leu Val Arg
       290                 295                 300
   Lys Asp Trp Phe Tyr Asp Glu Ile Phe Ala Gly Asp Thr His Ser Val
   305                 310                 315                 320
   Phe Tyr Glu Pro Arg Gln Asn Gly Lys Tyr Ser Phe Asn Asp Asp Asn
                   325                 330                 335
   Asn Gly Thr Gly Lys Ile Asn Ala Lys His Glu His Asn Ser Leu Pro
               340                 345                 350
   Asn Arg Leu Lys Thr Arg Thr Val Gln Leu Phe Asn Val Ser Leu Ser
           355                 360                 365
   Glu Thr Ala Arg Glu Pro Val Tyr His Ala Ala Gly Gly Val Asn Ser
       370                 375                 380
   Tyr Arg Pro Arg Leu Asn Asn Gly Glu Asn Ile Ser Phe Ile Asp Glu
   385                 390                 395                 400
   Gly Lys Gly Glu Leu Ile Leu Thr Ser Asn Ile Asn Gln Gly Ala Gly
   405 410 415
   Gly Leu Tyr Phe Gln Gly Asp Phe Thr Val Ser Pro Glu Asn Asn Glu
   420 425 430
   Thr Trp Gln Gly Ala Gly Val His Ile Ser Glu Asp Ser Thr Val Thr
   435 440 445
   Trp Lys Val Asn Gly Val Ala Asn Asp Arg Leu Ser Lys Ile Gly Lys
   450 455 460
   Gly Thr Leu His Val Gln Ala Lys Gly Glu Asn Gln Gly Ser Ile Ser
   465 470 475 480
   Val Gly Asp Gly Thr Val Ile Leu Asp Gln Gln Ala Asp Asp Lys Gly
   485 490 495
   Lys Lys Gln Ala Phe Ser Glu Ile Gly Leu Val Ser Gly Arg Gly Thr
   500 505 510
   Val Gln Leu Asn Ala Asp Asn Gln Phe Asn Pro Asp Lys Leu Tyr Phe
   515 520 525
   Gly Phe Arg Gly Gly Arg Leu Asp Leu Asn Gly His Ser Leu Ser Phe
   530 535 540
   His Arg Ile Gln Asn Thr Asp Glu Gly Ala Met Ile Val Asn His Asn
   545 550 555 560
   Gln Asp Lys Glu Ser Thr Val Thr Ile Thr Gly Asn Lys Asp Ile Ala
   565 570 575
   Thr Thr Gly Asn Asn Asn Ser Leu Asp Ser Lys Lys Glu Ile Ala Tyr
   580 585 590
   Asn Gly Trp Phe Gly Glu Lys Asp Thr Thr Lys Thr Asn Gly Arg Leu
   595 600 605
   Asn Leu Val Tyr Gln Pro Ala Ala Glu Asp Arg Thr Leu Leu Leu Ser
   610 615 620
   Gly Gly Thr Asn Leu Asn Gly Asn Ile Thr Gln Thr Asn Gly Lys Leu
   625 630 635 640
   Phe Phe Ser Gly Arg Pro Thr Pro His Ala Tyr Asn His Leu Asn Asp
   645 650 655
   His Trp Ser Gln Lys Glu Gly Ile Pro Arg Gly Glu Ile Val Trp Asp
   660 665 670
   Asn Asp Trp Ile Asn Arg Thr Phe Lys Ala Glu Asn Phe Gln Ile Lys
   675 680 685
   Gly Gly Gln Ala Val Val Ser Arg Asn Val Ala Lys Val Lys Gly Asp
   690 695 700
   Trp His Leu Ser Asn His Ala Gln Ala Val Phe Gly Val Ala Pro His
   705 710 715 720
   Gln Ser His Thr Ile Cys Thr Arg Ser Asp Trp Thr Gly Leu Thr Asn
   725 730 735
   Cys Val Glu Lys Thr Ile Thr Asp Asp Lys Val Ile Ala Ser Leu Thr
               740                 745                 750
   Lys Thr Asp Ile Ser Gly Asn Val Asp Leu Ala Asp His Ala His Leu
           755                 760                 765
   Asn Leu Thr Gly Leu Ala Thr Leu Asn Gly Asn Leu Ser Ala Asn Gly
       770                 775                 780
   Asp Thr Arg Tyr Thr Val Ser His Asn Ala Thr Gln Asn Gly Asn Leu
   785                 790                 795                 800
   Ser Leu Val Gly Asn Ala Gln Ala Thr Phe Asn Gln Ala Thr Leu Asn
                   805                 810                 815
   Gly Asn Thr Ser Ala Ser Gly Asn Ala Ser Phe Asn Leu Ser Asp His
               820                 825                 830
   Ala Val Gln Asn Gly Ser Leu Thr Leu Ser Gly Asn Ala Lys Ala Asn
           835                 840                 845
   Val Ser His Ser Ala Leu Asn Gly Asn Val Ser Leu Ala Asp Lys Ala
       850                 855                 860
   Val Phe His Phe Glu Ser Ser Arg Phe Thr Gly Gln Ile Ser Gly Gly
   865                 870                 875                 880
   Lys Asp Thr Ala Leu His Leu Lys Asp Ser Glu Trp Thr Leu Pro Ser
                   885                 890                 895
   Gly Thr Glu Leu Gly Asn Leu Asn Leu Asp Asn Ala Thr Ile Thr Leu
               900                 905                 910
   Asn Ser Ala Tyr Arg His Asp Ala Ala Gly Ala Gln Thr Gly Ser Ala
           915                 920                 925
   Thr Asp Ala Pro Arg Arg Arg Ser Arg Arg Ser Arg Arg Ser Leu Leu
       930                 935                 940
   Ser Val Thr Pro Pro Thr Ser Val Glu Ser Arg Phe Asn Thr Leu Thr
   945                 950                 955                 960
   Val Asn Gly Lys Leu Asn Gly Gln Gly Thr Phe Arg Phe Met Ser Glu
                   965                 970                 975
   Leu Phe Gly Tyr Arg Ser Asp Lys Leu Lys Leu Ala Glu Ser Ser Glu
               980                 985                 990
   Gly Thr Tyr Thr Leu Ala Val Asn Asn Thr Gly Asn Glu Pro Ala Ser
           995                 1000                1005
   Leu Glu Gln Leu Thr Val Val Glu Gly Lys Asp Asn Lys Pro Leu Ser
       1010                1015                1020
   Glu Asn Leu Asn Phe Thr Leu Gln Asn Glu His Val Asp Ala Gly Ala
   1025                1030                1035                1040
   Trp Arg Tyr Gln Leu Ile Arg Lys Asp Gly Glu Phe Arg Leu His Asn
                   1045                1050                1055
   Pro Val Lys Glu Gln Glu Leu Ser Asp Lys Leu Gly Lys Ala Glu Ala
               1060                1065                1070
   Lys Lys Gln Ala Glu Lys Asp Asn Ala Gln Ser Leu Asp Ala Leu Ile
   1075 1080 1085
   Ala Ala Gly Arg Asp Ala Val Glu Lys Thr Glu Ser Val Ala Glu Pro
   1090 1095 1100
   Ala Arg Gln Ala Gly Gly Glu Asn Val Gly Ile Met Gln Ala Glu Glu
   1105 1110 1115 1120
   Glu Lys Lys Arg Val Gln Ala Asp Lys Asp Thr Ala Leu Ala Lys Gln
   1125 1130 1135
   Arg Glu Ala Glu Thr Arg Pro Ala Thr Thr Ala Phe Pro Arg Ala Arg
   1140 1145 1150
   Arg Ala Arg Arg Asp Leu Pro Gln Leu Gln Pro Gln Pro Gln Pro Gln
   1155 1160 1165
   Pro Gln Arg Asp Leu Ile Ser Arg Tyr Ala Asn Ser Gly Leu Ser Glu
   1170 1175 1180
   Phe Ser Ala Thr Leu Asn Ser Val Phe Ala Val Gln Asp Glu Leu Asp
   1185 1190 1195 1200
   Arg Val Phe Ala Glu Asp Arg Arg Asn Ala Val Trp Thr Ser Gly Ile
   1205 1210 1215
   Arg Asp Thr Lys His Tyr Arg Ser Gln Asp Phe Arg Ala Tyr Arg Gln
   1220 1225 1230
   Gln Thr Asp Leu Arg Gln Ile Gly Met Gln Lys Asn Leu Gly Ser Gly
   1235 1240 1245
   Arg Val Gly Ile Leu Phe Ser His Asn Arg Thr Glu Asn Thr Phe Asp
   1250 1255 1260
   Asp Gly Ile Gly Asn Ser Ala Arg Leu Ala His Gly Ala Val Phe Gly
   1265 1270 1275 1280
   Gln Tyr Gly Ile Asp Arg Phe Tyr Ile Gly Ile Ser Ala Gly Ala Gly
   1285 1290 1295
   Phe Ser Ser Gly Ser Leu Ser Asp Gly Ile Gly Gly Lys Ile Arg Arg
   1300 1305 1310
   Arg Val Leu His Tyr Gly Ile Gln Ala Arg Tyr Arg Ala Gly Phe Gly
   1315 1320 1325
   Gly Phe Gly Ile Glu Pro His Ile Gly Ala Thr Arg Tyr Phe Val Gln
   1330 1335 1340
   Lys Ala Asp Tyr Arg Tyr Glu Asn Val Asn Ile Ala Thr Pro Gly Leu
   1345 1350 1355 1360
   Ala Phe Asn Arg Tyr Arg Ala Gly Ile Lys Ala Asp Tyr Ser Phe Lys
   1365 1370 1375
   Pro Ala Gln His Ile Ser Ile Thr Pro Tyr Leu Ser Leu Ser Tyr Thr
   1380 1385 1390
   Asp Ala Ala Ser Gly Lys Val Arg Thr Arg Val Asn Thr Ala Val Leu
   1395 1400 1405
   Ala Gln Asp Phe Gly Lys Thr Arg Ser Ala Glu Trp Gly Val Asn Ala
       1410                1415                1420
   Glu Ile Lys Gly Phe Thr Leu Ser Leu His Ala Ala Ala Ala Lys Gly
   1425                1430                1435                1440
   Pro Gln Leu Glu Ala Gln His Ser Ala Gly Ile Lys Leu Gly Tyr Arg
                   1445                1450                1455
   Trp
<210> 14
   <211> 797
   <212> PRT
   <213> Neisseria meningitidis
<400> 14
   Met Lys Leu Lys Gln Ile Ala Ser Ala Leu Met Met Leu Gly Ile Ser
   1               5                   10                  15
   Pro Leu Ala Leu Ala Asp Phe Thr Ile Gln Asp Ile Arg Val Glu Gly
               20                  25                  30
   Leu Gln Arg Thr Glu Pro Ser Thr Val Phe Asn Tyr Leu Pro Val Lys
           35                  40                  45
   Val Gly Asp Thr Tyr Asn Asp Thr His Gly Ser Ala Ile Ile Lys Ser
       50                  55                  60
   Leu Tyr Ala Thr Gly Phe Phe Asp Asp Val Arg Val Glu Thr Ala Asp
   65                  70                  75                  80
   Gly Gln Leu Leu Leu Thr Val Ile Glu Arg Pro Thr Ile Gly Ser Leu
                   85                  90                  95
   Asn Ile Thr Gly Ala Lys Met Leu Gln Asn Asp Ala Ile Lys Lys Asn
               100                 105                 110
   Leu Glu Ser Phe Gly Leu Ala Gln Ser Gln Tyr Phe Asn Gln Ala Thr
           115                 120                 125
   Leu Asn Gln Ala Val Ala Gly Leu Lys Glu Glu Tyr Leu Gly Arg Gly
       130                 135                 140
   Lys Leu Asn Ile Gln Ile Thr Pro Lys Val Thr Lys Leu Ala Arg Asn
   145                 150                 155                 160
   Arg Val Asp Ile Asp Ile Thr Ile Asp Glu Gly Lys Ser Ala Lys Ile
                   165                 170                 175
   Thr Asp Ile Glu Phe Glu Gly Asn Gln Val Tyr Ser Asp Arg Lys Leu
               180                 185                 190
   Met Arg Gln Met Ser Leu Thr Glu Gly Gly Ile Trp Thr Trp Leu Thr
           195                 200                 205
   Arg Ser Asn Gln Phe Asn Glu Gln Lys Phe Ala Gln Asp Met Glu Lys
       210                 215                 220
   Val Thr Asp Phe Tyr Gln Asn Asn Gly Tyr Phe Asp Phe Arg Ile Leu
   225                 230                 235                 240
   Asp Thr Asp Ile Gln Thr Asn Glu Asp Lys Thr Lys Gln Thr Ile Lys
   245 250 255
   Ile Thr Val His Glu Gly Gly Arg Phe Arg Trp Gly Lys Val Ser Ile
   260 265 270
   Glu Gly Asp Thr Asn Glu Val Pro Lys Ala Glu Leu Glu Lys Leu Leu
   275 280 285
   Thr Met Lys Pro Gly Lys Trp Tyr Glu Arg Gln Gln Met Thr Ala Val
   290 295 300
   Leu Gly Glu Ile Gln Asn Arg Met Gly Ser Ala Gly Tyr Ala Tyr Ser
   305 310 315 320
   Glu Ile Ser Val Gln Pro Leu Pro Asn Ala Glu Thr Lys Thr Val Asp
   325 330 335
   Phe Val Leu His Ile Glu Pro Gly Arg Lys Ile Tyr Val Asn Glu Ile
   340 345 350
   His Ile Thr Gly Asn Asn Lys Thr Arg Asp Glu Val Val Arg Arg Glu
   355 360 365
   Leu Arg Gln Met Glu Ser Ala Pro Tyr Asp Thr Ser Lys Leu Gln Arg
   370 375 380
   Ser Lys Glu Arg Val Glu Leu Leu Gly Tyr Phe Asp Asn Val Gln Phe
   385 390 395 400
   Asp Ala Val Pro Leu Ala Gly Thr Pro Asp Lys Val Asp Leu Asn Met
   405 410 415
   Ser Leu Thr Glu Arg Ser Thr Gly Ser Leu Asp Leu Ser Ala Gly Trp
   420 425 430
   Val Gln Asp Thr Gly Leu Val Met Ser Ala Gly Val Ser Gln Asp Asn
   435 440 445
   Leu Phe Gly Thr Gly Lys Ser Ala Ala Leu Arg Ala Ser Arg Ser Lys
   450 455 460
   Thr Thr Leu Asn Gly Ser Leu Ser Phe Thr Asp Pro Tyr Phe Thr Ala
   465 470 475 480
   Asp Gly Val Ser Leu Gly Tyr Asp Val Tyr Gly Lys Ala Phe Asp Pro
   485 490 495
   Arg Lys Ala Ser Thr Ser Ile Lys Gln Tyr Lys Thr Thr Thr Ala Gly
   500 505 510
   Ala Gly Ile Arg Met Ser Val Pro Val Thr Glu Tyr Asp Arg Val Asn
   515 520 525
   Phe Gly Leu Val Ala Glu His Leu Thr Val Asn Thr Tyr Asn Lys Ala
   530 535 540
   Pro Lys His Tyr Ala Asp Phe Ile Lys Lys Tyr Gly Lys Thr Asp Gly
   545 550 555 560
   Thr Asp Gly Ser Phe Lys Gly Trp Leu Tyr Lys Gly Thr Val Gly Trp
   565 570 575
   Gly Arg Asn Lys Thr Asp Ser Ala Leu Trp Pro Thr Arg Gly Tyr Leu
                   580                 585                 590
   Thr Gly Val Asn Ala Glu Ile Ala Leu Pro Gly Ser Lys Leu Gln Tyr
           595                 600                 605
   Tyr Ser Ala Thr His Asn Gln Thr Trp Phe Phe Pro Leu Ser Lys Thr
       610                 615                 620
   Phe Thr Leu Met Leu Gly Gly Glu Val Gly Ile Ala Gly Gly Tyr Gly
   625                 630                 635                 640
   Arg Thr Lys Glu Ile Pro Phe Phe Glu Asn Phe Tyr Gly Gly Gly Leu
                   645                 650                 655
   Gly Ser Val Arg Gly Tyr Glu Ser Gly Thr Leu Gly Pro Lys Val Tyr
               660                 665                 670
   Asp Glu Tyr Gly Glu Lys Ile Ser Tyr Gly Gly Asn Lys Lys Ala Asn
           675                 680                 685
   Val Ser Ala Glu Leu Leu Phe Pro Met Pro Gly Ala Lys Asp Ala Arg
       690                 695                 700
   Thr Val Arg Leu Ser Leu Phe Ala Asp Ala Gly Ser Val Trp Asp Gly
   705                 710                 715                 720
   Lys Thr Tyr Asp Asp Asn Ser Ser Ser Ala Thr Gly Gly Arg Val Gln
                   725                 730                 735
   Asn Ile Tyr Gly Ala Gly Asn Thr His Lys Ser Thr Phe Thr Asn Glu
               740                 745                 750
   Leu Arg Tyr Ser Ala Gly Gly Ala Val Thr Trp Leu Ser Pro Leu Gly
           755                 760                 765
   Pro Met Lys Phe Ser Tyr Ala Tyr Pro Leu Lys Lys Lys Pro Glu Asp
       770                 775                 780
   Glu Ile Gln Arg Phe Gln Phe Gln Leu Gly Thr Thr Phe
   785                 790                 795
<210> 15
   <211> 6
   <212> PRT
   <213> Artificial Sequence
<220>
   <223> Linker
<400> 15
   Gly Ser Gly Gly Gly Gly
   1               5
<210> 16
   <211> 8
   <212> PRT
   <213> Artificial Sequence
<220>
   <223> Linker
<400> 16
   Gly Ser Gly Ser Gly Gly Gly Gly
   1 5
<210> 17
   <211> 18
   <212> DNA
   <213> Neisseria meningitidis
<400> 17
   tttgcggggg gggggggg 18
<210> 18
   <211> 35
   <212> DNA
   <213> Neisseria meningitidis
<400> 18
   ttgacaggcg gaaggaatac tttataattc gcaac 35
<210> 19
   <211> 791
   <212> PRT
   <213> Neisseria meningitidis
<400> 19
   Met Lys Pro Leu Gln Met Leu Pro Ile Ala Ala Leu Val Gly Ser Ile
   1               5                   10                  15
   Phe Gly Asn Pro Val Leu Ala Ala Asp Glu Ala Ala Thr Glu Thr Thr
               20                  25                  30
   Pro Val Lys Ala Glu Ile Lys Ala Val Arg Val Lys Gly Gln Arg Asn
           35                  40                  45
   Ala Pro Ala Ala Val Glu Arg Val Asn Leu Asn Arg Ile Lys Gln Glu
       50                  55                  60
   Met Ile Arg Asp Asn Lys Asp Leu Val Arg Tyr Ser Thr Asp Val Gly
   65                  70                  75                  80
   Leu Ser Asp Ser Gly Arg His Gln Lys Gly Phe Ala Val Arg Gly Val
                   85                  90                  95
   Glu Gly Asn Arg Val Gly Val Ser Ile Asp Gly Val Asn Leu Pro Asp
               100                 105                 110
   Ser Glu Glu Asn Ser Leu Tyr Ala Arg Tyr Gly Asn Phe Asn Ser Ser
           115                 120                 125
   Arg Leu Ser Ile Asp Pro Glu Leu Val Arg Asn Ile Glu Ile Val Lys
       130                 135                 140
   Gly Ala Asp Ser Phe Asn Thr Gly Ser Gly Ala Leu Gly Gly Gly Val
   145                 150                 155                 160
   Asn Tyr Gln Thr Leu Gln Gly Arg Asp Leu Leu Leu Asp Asp Arg Gln
                   165                 170                 175
   Phe Gly Val Met Met Lys Asn Gly Tyr Ser Thr Arg Asn Arg Glu Trp
               180                 185                 190
   Thr Asn Thr Leu Gly Phe Gly Val Ser Asn Asp Arg Val Asp Ala Ala
           195                 200                 205
   Leu Leu Tyr Ser Gln Arg Arg Gly His Glu Thr Glu Ser Ala Gly Asn
       210                 215                 220
   Arg Gly Tyr Ala Val Glu Gly Glu Gly Ser Gly Ala Asn Ile Arg Gly
   225                 230                 235                 240
   Ser Ala Arg Gly Ile Pro Asp Ser Ser Lys His Lys Tyr His Ser Phe
                   245                 250                 255
   Leu Gly Lys Ile Ala Tyr Gln Ile Asn Asp Asn His Arg Ile Gly Ala
               260                 265                 270
   Ser Leu Asn Gly Gln Gln Gly His Asn Tyr Thr Val Glu Glu Ser Tyr
           275                 280                 285
   Asn Leu Thr Ala Ser Ser Trp Arg Glu Ala Asp Asp Val Asn Arg Arg
       290                 295                 300
   Arg Asn Ala Asn Leu Phe Tyr Glu Trp Met Pro Asp Ser Asn Trp Leu
   305                 310                 315                 320
   Ser Ser Leu Lys Ala Asp Phe Asp Tyr Gln Lys Thr Lys Val Ala Ala
                   325                 330                 335
   Val Asn Asn Lys Gly Ser Phe Pro Met Asp Tyr Ser Thr Trp Thr Arg
               340                 345                 350
   Asn Tyr Asn Gln Lys Asp Leu Asp Glu Ile Tyr Asn Arg Ser Met Asp
           355                 360                 365
   Thr Arg Phe Lys Arg Phe Thr Leu Arg Leu Asp Ser His Pro Leu Gln
       370                 375                 380
   Leu Gly Gly Gly Arg His Arg Leu Ser Phe Lys Thr Phe Val Ser Arg
   385                 390                 395                 400
   Arg Asp Phe Glu Asn Leu Asn Arg Asp Asp Tyr Tyr Phe Ser Gly Arg
                   405                 410                 415
   Val Val Arg Thr Thr Ser Ser Ile Gln His Pro Val Lys Thr Thr Asn
               420                 425                 430
   Tyr Gly Phe Ser Leu Ser Asp Gln Ile Gln Trp Asn Asp Val Phe Ser
           435                 440                 445
   Ser Arg Ala Gly Ile Arg Tyr Asp His Thr Lys Met Thr Pro Gln Glu
       450                 455                 460
   Leu Asn Ala Glu Cys His Ala Cys Asp Lys Thr Pro Pro Ala Ala Asn
   465                 470                 475                 480
   Thr Tyr Lys Gly Trp Ser Gly Phe Val Gly Leu Ala Ala Gln Leu Asn
                   485                 490                 495
   Gln Ala Trp His Val Gly Tyr Asp Ile Thr Ser Gly Tyr Arg Val Pro
               500                 505                 510
   Asn Ala Ser Glu Val Tyr Phe Thr Tyr Asn His Gly Ser Gly Asn Trp
           515                 520                 525
   Leu Pro Asn Pro Asn Leu Lys Ala Glu Arg Ser Thr Thr His Thr Leu
       530                 535                 540
   Ser Leu Gln Gly Arg Ser Glu Lys Gly Met Leu Asp Ala Asn Leu Tyr
   545                 550                 555                 560
   Gln Ser Asn Tyr Arg Asn Phe Leu Ser Glu Glu Gln Lys Leu Thr Thr
                   565                 570                 575
   Ser Gly Thr Pro Gly Cys Thr Glu Glu Asn Ala Tyr Tyr Gly Ile Cys
               580                 585                 590
   Ser Asp Pro Tyr Lys Glu Lys Leu Asp Trp Gln Met Lys Asn Ile Asp
           595                 600                 605
   Lys Ala Arg Ile Arg Gly Ile Glu Leu Thr Gly Arg Leu Asn Val Asp
       610                 615                 620
   Lys Val Ala Ser Phe Val Pro Glu Gly Trp Lys Leu Phe Gly Ser Leu
   625                 630                 635                 640
   Gly Tyr Ala Lys Ser Lys Leu Ser Gly Asp Asn Ser Leu Leu Ser Thr
                   645                 650                 655
   Gln Pro Leu Lys Val Ile Ala Gly Ile Asp Tyr Glu Ser Pro Ser Glu
               660                 665                 670
   Lys Trp Gly Val Phe Ser Arg Leu Thr Tyr Leu Gly Ala Lys Lys Ala
           675                 680                 685
   Lys Asp Ala Gln Tyr Thr Val Tyr Glu Asn Lys Gly Trp Gly Thr Pro
       690                 695                 700
   Leu Gln Lys Lys Val Lys Asp Tyr Pro Trp Leu Asn Lys Ser Ala Tyr
   705                 710                 715                 720
   Val Phe Asp Met Tyr Gly Phe Tyr Lys Pro Ala Lys Asn Leu Thr Leu
                   725                 730                 735
   Arg Ala Gly Val Tyr Asn Val Phe Asn Arg Lys Tyr Thr Thr Trp Asp
               740                 745                 750
   Ser Leu Arg Gly Leu Tyr Ser Tyr Ser Thr Thr Asn Ser Val Asp Arg
           755                 760                 765
   Asp Gly Lys Gly Leu Asp Arg Tyr Arg Ala Pro Ser Arg Asn Tyr Ala
       770                 775                 780
   Val Ser Leu Glu Trp Lys Phe
   785                 790
<210> 20
   <211> 147
   <212> PRT
   <213> Neisseria meningitidis
<400> 20
   Ala Asp Glu Ala Ala Thr Glu Thr Thr Pro Val Lys Ala Glu Ile Lys
   1 5 10 15
   Ala Val Arg Val Lys Gly Gln Arg Asn Ala Pro Ala Ala Val Glu Arg
   20 25 30
   Val Asn Leu Asn Arg Ile Lys Gln Glu Met Ile Arg Asp Asn Lys Asp
   35 40 45
   Leu Val Arg Tyr Ser Thr Asp Val Gly Leu Ser Asp Ser Gly Arg His
   50 55 60
   Gln Lys Gly Phe Ala Val Arg Gly Val Glu Gly Asn Arg Val Gly Val
   65 70 75 80
   Ser Ile Asp Gly Val Asn Leu Pro Asp Ser Glu Glu Asn Ser Leu Tyr
   85 90 95
   Ala Arg Tyr Gly Asn Phe Asn Ser Ser Arg Leu Ser Ile Asp Pro Glu
   100 105 110
   Leu Val Arg Asn Ile Glu Ile Val Lys Gly Ala Asp Ser Phe Asn Thr
   115 120 125
   Gly Ser Gly Ala Leu Gly Gly Gly Val Asn Tyr Gln Thr Leu Gln Gly
   130 135 140
   Arg Asp Leu
   145
<210> 21
   <211> 621
   <212> PRT
   <213> Neisseria meningitidis
<400> 21
   Leu Leu Asp Asp Arg Gln Phe Gly Val Met Met Lys Asn Gly Tyr Ser
   1               5                   10                  15
   Thr Arg Asn Arg Glu Trp Thr Asn Thr Leu Gly Phe Gly Val Ser Asn
               20                  25                  30
   Asp Arg Val Asp Ala Ala Leu Leu Tyr Ser Gln Arg Arg Gly His Glu
           35                  40                  45
   Thr Glu Ser Ala Gly Asn Arg Gly Tyr Ala Val Glu Gly Glu Gly Ser
       50                  55                  60
   Gly Ala Asn Ile Arg Gly Ser Ala Arg Gly Ile Pro Asp Ser Ser Lys
   65                  70                  75                  80
   His Lys Tyr His Ser Phe Leu Gly Lys Ile Ala Tyr Gln Ile Asn Asp
                   85                  90                  95
   Asn His Arg Ile Gly Ala Ser Leu Asn Gly Gln Gln Gly His Asn Tyr
               100                 105                 110
   Thr Val Glu Glu Ser Tyr Asn Leu Thr Ala Ser Ser Trp Arg Glu Ala
           115                 120                 125
   Asp Asp Val Asn Arg Arg Arg Asn Ala Asn Leu Phe Tyr Glu Trp Met
       130                 135                 140
   Pro Asp Ser Asn Trp Leu Ser Ser Leu Lys Ala Asp Phe Asp Tyr Gln
   145                 150                 155                 160
   Lys Thr Lys Val Ala Ala Val Asn Asn Lys Gly Ser Phe Pro Met Asp
                   165                 170                 175
   Tyr Ser Thr Trp Thr Arg Asn Tyr Asn Gln Lys Asp Leu Asp Glu Ile
               180                 185                 190
   Tyr Asn Arg Ser Met Asp Thr Arg Phe Lys Arg Phe Thr Leu Arg Leu
   195 200 205
   Asp Ser His Pro Leu Gln Leu Gly Gly Gly Arg His Arg Leu Ser Phe
   210 215 220
   Lys Thr Phe Val Ser Arg Arg Asp Phe Glu Asn Leu Asn Arg Asp Asp
   225 230 235 240
   Tyr Tyr Phe Ser Gly Arg Val Val Arg Thr Thr Ser Ser Ile Gln His
   245 250 255
   Pro Val Lys Thr Thr Asn Tyr Gly Phe Ser Leu Ser Asp Gln Ile Gln
   260 265 270
   Trp Asn Asp Val Phe Ser Ser Arg Ala Gly Ile Arg Tyr Asp His Thr
   275 280 285
   Lys Met Thr Pro Gln Glu Leu Asn Ala Glu Cys His Ala Cys Asp Lys
   290 295 300
   Thr Pro Pro Ala Ala Asn Thr Tyr Lys Gly Trp Ser Gly Phe Val Gly
   305 310 315 320
   Leu Ala Ala Gln Leu Asn Gln Ala Trp His Val Gly Tyr Asp Ile Thr
   325 330 335
   Ser Gly Tyr Arg Val Pro Asn Ala Ser Glu Val Tyr Phe Thr Tyr Asn
   340 345 350
   His Gly Ser Gly Asn Trp Leu Pro Asn Pro Asn Leu Lys Ala Glu Arg
   355 360 365
   Ser Thr Thr His Thr Leu Ser Leu Gln Gly Arg Ser Glu Lys Gly Met
   370 375 380
   Leu Asp Ala Asn Leu Tyr Gln Ser Asn Tyr Arg Asn Phe Leu Ser Glu
   385 390 395 400
   Glu Gln Lys Leu Thr Thr Ser Gly Thr Pro Gly Cys Thr Glu Glu Asn
   405 410 415
   Ala Tyr Tyr Gly Ile Cys Ser Asp Pro Tyr Lys Glu Lys Leu Asp Trp
   420 425 430
   Gln Met Lys Asn Ile Asp Lys Ala Arg Ile Arg Gly Ile Glu Leu Thr
   435 440 445
   Gly Arg Leu Asn Val Asp Lys Val Ala Ser Phe Val Pro Glu Gly Trp
   450 455 460
   Lys Leu Phe Gly Ser Leu Gly Tyr Ala Lys Ser Lys Leu Ser Gly Asp
   465 470 475 480
   Asn Ser Leu Leu Ser Thr Gln Pro Leu Lys Val Ile Ala Gly Ile Asp
   485 490 495
   Tyr Glu Ser Pro Ser Glu Lys Trp Gly Val Phe Ser Arg Leu Thr Tyr
   500 505 510
   Leu Gly Ala Lys Lys Ala Lys Asp Ala Gln Tyr Thr Val Tyr Glu Asn
   515 520 525
   Lys Gly Trp Gly Thr Pro Leu Gln Lys Lys Val Lys Asp Tyr Pro Trp
       530                 535                 540
   Leu Asn Lys Ser Ala Tyr Val Phe Asp Met Tyr Gly Phe Tyr Lys Pro
   545                 550                 555                 560
   Ala Lys Asn Leu Thr Leu Arg Ala Gly Val Tyr Asn Val Phe Asn Arg
                   565                 570                 575
   Lys Tyr Thr Thr Trp Asp Ser Leu Arg Gly Leu Tyr Ser Tyr Ser Thr
               580                 585                 590
   Thr Asn Ser Val Asp Arg Asp Gly Lys Gly Leu Asp Arg Tyr Arg Ala
           595                 600                 605
   Pro Ser Arg Asn Tyr Ala Val Ser Leu Glu Trp Lys Phe
       610                 615                 620
<210> 22
   <211> 768
   <212> PRT
   <213> Neisseria meningitidis
<400> 22
   Ala Asp Glu Ala Ala Thr Glu Thr Thr Pro Val Lys Ala Glu Ile Lys
   1               5                   10                  15
   Ala Val Arg Val Lys Gly Gln Arg Asn Ala Pro Ala Ala Val Glu Arg
               20                  25                  30
   Val Asn Leu Asn Arg Ile Lys Gln Glu Met Ile Arg Asp Asn Lys Asp
           35                  40                  45
   Leu Val Arg Tyr Ser Thr Asp Val Gly Leu Ser Asp Ser Gly Arg His
       50                  55                  60
   Gln Lys Gly Phe Ala Val Arg Gly Val Glu Gly Asn Arg Val Gly Val
   65                  70                  75                  80
   Ser Ile Asp Gly Val Asn Leu Pro Asp Ser Glu Glu Asn Ser Leu Tyr
                   85                  90                  95
   Ala Arg Tyr Gly Asn Phe Asn Ser Ser Arg Leu Ser Ile Asp Pro Glu
               100                 105                 110
   Leu Val Arg Asn Ile Glu Ile Val Lys Gly Ala Asp Ser Phe Asn Thr
           115                 120                 125
   Gly Ser Gly Ala Leu Gly Gly Gly Val Asn Tyr Gln Thr Leu Gln Gly
       130                 135                 140
   Arg Asp Leu Leu Leu Asp Asp Arg Gln Phe Gly Val Met Met Lys Asn
   145                 150                 155                 160
   Gly Tyr Ser Thr Arg Asn Arg Glu Trp Thr Asn Thr Leu Gly Phe Gly
                   165                 170                 175
   Val Ser Asn Asp Arg Val Asp Ala Ala Leu Leu Tyr Ser Gln Arg Arg
               180                 185                 190
   Gly His Glu Thr Glu Ser Ala Gly Asn Arg Gly Tyr Ala Val Glu Gly
           195                 200                 205
   Glu Gly Ser Gly Ala Asn Ile Arg Gly Ser Ala Arg Gly Ile Pro Asp
       210                 215                 220
   Ser Ser Lys His Lys Tyr His Ser Phe Leu Gly Lys Ile Ala Tyr Gln
   225                 230                 235                 240
   Ile Asn Asp Asn His Arg Ile Gly Ala Ser Leu Asn Gly Gln Gln Gly
                   245                 250                 255
   His Asn Tyr Thr Val Glu Glu Ser Tyr Asn Leu Thr Ala Ser Ser Trp
               260                 265                 270
   Arg Glu Ala Asp Asp Val Asn Arg Arg Arg Asn Ala Asn Leu Phe Tyr
           275                 280                 285
   Glu Trp Met Pro Asp Ser Asn Trp Leu Ser Ser Leu Lys Ala Asp Phe
       290                 295                 300
   Asp Tyr Gln Lys Thr Lys Val Ala Ala Val Asn Asn Lys Gly Ser Phe
   305                 310                 315                 320
   Pro Met Asp Tyr Ser Thr Trp Thr Arg Asn Tyr Asn Gln Lys Asp Leu
                   325                 330                 335
   Asp Glu Ile Tyr Asn Arg Ser Met Asp Thr Arg Phe Lys Arg Phe Thr
               340                 345                 350
   Leu Arg Leu Asp Ser His Pro Leu Gln Leu Gly Gly Gly Arg His Arg
           355                 360                 365
   Leu Ser Phe Lys Thr Phe Val Ser Arg Arg Asp Phe Glu Asn Leu Asn
       370                 375                 380
   Arg Asp Asp Tyr Tyr Phe Ser Gly Arg Val Val Arg Thr Thr Ser Ser
   385                 390                 395                 400
   Ile Gln His Pro Val Lys Thr Thr Asn Tyr Gly Phe Ser Leu Ser Asp
                   405                 410                 415
   Gln Ile Gln Trp Asn Asp Val Phe Ser Ser Arg Ala Gly Ile Arg Tyr
               420                 425                 430
   Asp His Thr Lys Met Thr Pro Gln Glu Leu Asn Ala Glu Cys His Ala
           435                 440                 445
   Cys Asp Lys Thr Pro Pro Ala Ala Asn Thr Tyr Lys Gly Trp Ser Gly
       450                 455                 460
   Phe Val Gly Leu Ala Ala Gln Leu Asn Gln Ala Trp His Val Gly Tyr
   465                 470                 475                 480
   Asp Ile Thr Ser Gly Tyr Arg Val Pro Asn Ala Ser Glu Val Tyr Phe
                   485                 490                 495
   Thr Tyr Asn His Gly Ser Gly Asn Trp Leu Pro Asn Pro Asn Leu Lys
               500                 505                 510
   Ala Glu Arg Ser Thr Thr His Thr Leu Ser Leu Gln Gly Arg Ser Glu
           515                 520                 525
   Lys Gly Met Leu Asp Ala Asn Leu Tyr Gln Ser Asn Tyr Arg Asn Phe
       530                 535                 540
   Leu Ser Glu Glu Gln Lys Leu Thr Thr Ser Gly Thr Pro Gly Cys Thr
   545 550 555 560
   Glu Glu Asn Ala Tyr Tyr Gly Ile Cys Ser Asp Pro Tyr Lys Glu Lys
   565 570 575
   Leu Asp Trp Gln Met Lys Asn Ile Asp Lys Ala Arg Ile Arg Gly Ile
   580 585 590
   Glu Leu Thr Gly Arg Leu Asn Val Asp Lys Val Ala Ser Phe Val Pro
   595 600 605
   Glu Gly Trp Lys Leu Phe Gly Ser Leu Gly Tyr Ala Lys Ser Lys Leu
   610 615 620
   Ser Gly Asp Asn Ser Leu Leu Ser Thr Gln Pro Leu Lys Val Ile Ala
   625 630 635 640
   Gly Ile Asp Tyr Glu Ser Pro Ser Glu Lys Trp Gly Val Phe Ser Arg
   645 650 655
   Leu Thr Tyr Leu Gly Ala Lys Lys Ala Lys Asp Ala Gln Tyr Thr Val
   660 665 670
   Tyr Glu Asn Lys Gly Trp Gly Thr Pro Leu Gln Lys Lys Val Lys Asp
   675 680 685
   Tyr Pro Trp Leu Asn Lys Ser Ala Tyr Val Phe Asp Met Tyr Gly Phe
   690 695 700
   Tyr Lys Pro Ala Lys Asn Leu Thr Leu Arg Ala Gly Val Tyr Asn Val
   705 710 715 720
   Phe Asn Arg Lys Tyr Thr Thr Trp Asp Ser Leu Arg Gly Leu Tyr Ser
   725 730 735
   Tyr Ser Thr Thr Asn Ser Val Asp Arg Asp Gly Lys Gly Leu Asp Arg
   740 745 750
   Tyr Arg Ala Pro Ser Arg Asn Tyr Ala Val Ser Leu Glu Trp Lys Phe
   755 760 765
<210> 23
   <211> 915
   <212> PRT
   <213> Neisseria meningitidis
<400> 23
   Met Gln Gln Gln His Leu Phe Arg Phe Asn Ile Leu Cys Leu Ser Leu
   1 5 10 15
   Met Thr Ala Leu Pro Ala Tyr Ala Glu Asn Val Gln Ala Gly Gln Ala
   20 25 30
   Gln Glu Lys Gln Leu Asp Thr Ile Gln Val Lys Ala Lys Lys Gln Lys
   35 40 45
   Thr Arg Arg Asp Asn Glu Val Thr Gly Leu Gly Lys Leu Val Lys Ser
   50 55 60
   Ser Asp Thr Leu Ser Lys Glu Gln Val Leu Asn Ile Arg Asp Leu Thr
   65                  70                  75                  80
   Arg Tyr Asp Pro Gly Ile Ala Val Val Glu Gln Gly Arg Gly Ala Ser
                   85                  90                  95
   Ser Gly Tyr Ser Ile Arg Gly Met Asp Lys Asn Arg Val Ser Leu Thr
               100                 105                 110
   Val Asp Gly Val Ser Gln Ile Gln Ser Tyr Thr Ala Gln Ala Ala Leu
           115                 120                 125
   Gly Gly Thr Arg Thr Ala Gly Ser Ser Gly Ala Ile Asn Glu Ile Glu
       130                 135                 140
   Tyr Glu Asn Val Lys Ala Val Glu Ile Ser Lys Gly Ser Asn Ser Val
   145                 150                 155                 160
   Glu Gln Gly Ser Gly Ala Leu Ala Gly Ser Val Ala Phe Gln Thr Lys
                   165                 170                 175
   Thr Ala Asp Asp Val Ile Gly Glu Gly Arg Gln Trp Gly Ile Gln Ser
               180                 185                 190
   Lys Thr Ala Tyr Ser Gly Lys Asn Arg Gly Leu Thr Gln Ser Ile Ala
           195                 200                 205
   Leu Ala Gly Arg Ile Gly Gly Ala Glu Ala Leu Leu Ile His Thr Gly
       210                 215                 220
   Arg Arg Ala Gly Glu Ile Arg Ala His Glu Asp Ala Gly Arg Gly Val
   225                 230                 235                 240
   Gln Ser Phe Asn Arg Leu Val Pro Val Glu Asp Ser Ser Asn Tyr Ala
                   245                 250                 255
   Tyr Phe Ile Val Lys Glu Glu Cys Lys Asn Gly Ser Tyr Glu Thr Cys
               260                 265                 270
   Lys Ala Asn Pro Lys Lys Asp Val Val Gly Lys Asp Glu Arg Gln Thr
           275                 280                 285
   Val Ser Thr Arg Asp Tyr Thr Gly Pro Asn Arg Phe Leu Ala Asp Pro
       290                 295                 300
   Leu Ser Tyr Glu Ser Arg Ser Trp Leu Phe Arg Pro Gly Phe Arg Phe
   305                 310                 315                 320
   Glu Asn Lys Arg His Tyr Ile Gly Gly Ile Leu Glu His Thr Gln Gln
                   325                 330                 335
   Thr Phe Asp Thr Arg Asp Met Thr Val Pro Ala Phe Leu Thr Lys Ala
               340                 345                 350
   Val Phe Asp Ala Asn Lys Lys Gln Ala Gly Ser Leu Pro Gly Asn Gly
           355                 360                 365
   Lys Tyr Ala Gly Asn His Lys Tyr Gly Gly Leu Phe Thr Asn Gly Glu
       370                 375                 380
   Asn Gly Ala Leu Val Gly Ala Glu Tyr Gly Thr Gly Val Phe Tyr Asp
   385                 390                 395                 400
   Glu Thr His Thr Lys Ser Arg Tyr Gly Leu Glu Tyr Val Tyr Thr Asn
                   405                 410                 415
   Ala Asp Lys Asp Thr Trp Ala Asp Tyr Ala Arg Leu Ser Tyr Asp Arg
               420                 425                 430
   Gln Gly Ile Gly Leu Asp Asn His Phe Gln Gln Thr His Cys Ser Ala
           435                 440                 445
   Asp Gly Ser Asp Lys Tyr Cys Arg Pro Ser Ala Asp Lys Pro Phe Ser
       450                 455                 460
   Tyr Tyr Lys Ser Asp Arg Val Ile Tyr Gly Glu Ser His Arg Leu Leu
   465                 470                 475                 480
   Gln Ala Ala Phe Lys Lys Ser Phe Asp Thr Ala Lys Ile Arg His Asn
                   485                 490                 495
   Leu Ser Val Asn Leu Gly Phe Asp Arg Phe Gly Ser Asn Leu Arg His
               500                 505                 510
   Gln Asp Tyr Tyr Tyr Gln His Ala Asn Arg Ala Tyr Ser Ser Asn Thr
           515                 520                 525
   Pro Pro Gln Asn Asn Gly Lys Lys Ile Ser Pro Asn Gly Ser Glu Thr
       530                 535                 540
   Ser Pro Tyr Trp Val Thr Ile Gly Arg Gly Asn Val Val Thr Gly Gln
   545                 550                 555                 560
   Ile Cys Arg Leu Gly Asn Asn Thr Tyr Thr Asp Cys Thr Pro Arg Ser
                   565                 570                 575
   Ile Asn Gly Lys Ser Tyr Tyr Ala Ala Val Arg Asp Asn Val Arg Leu
               580                 585                 590
   Gly Arg Trp Ala Asp Val Gly Ala Gly Leu Arg Tyr Asp Tyr Arg Ser
           595                 600                 605
   Thr His Ser Asp Asp Gly Ser Val Ser Thr Gly Thr His Arg Thr Leu
       610                 615                 620
   Ser Trp Asn Ala Gly Ile Val Leu Lys Pro Thr Asp Trp Leu Asp Leu
   625                 630                 635                 640
   Thr Tyr Arg Thr Ser Thr Gly Phe Arg Leu Pro Ser Phe Ala Glu Met
                   645                 650                 655
   Tyr Gly Trp Arg Ala Gly Val Gln Ser Lys Ala Val Lys Ile Asp Pro
               660                 665                 670
   Glu Lys Ser Phe Asn Lys Glu Ala Gly Ile Val Phe Lys Gly Asp Phe
           675                 680                 685
   Gly Asn Leu Glu Ala Ser Trp Phe Asn Asn Ala Tyr Arg Asp Leu Ile
       690                 695                 700
   Val Arg Gly Tyr Glu Ala Gln Ile Lys Asp Gly Lys Glu Glu Ala Lys
   705                 710                 715                 720
   Gly Asp Pro Ala Tyr Leu Asn Ala Gln Ser Ala Arg Ile Thr Gly Ile
                   725                 730                 735
   Asn Ile Leu Gly Lys Ile Asp Trp Asn Gly Val Trp Asp Lys Leu Pro
               740                 745                 750
   Glu Gly Trp Tyr Ser Thr Phe Ala Tyr Asn Arg Val Arg Val Arg Asp
           755                 760                 765
   Ile Lys Lys Arg Ala Asp Arg Thr Asp Ile Gln Ser His Leu Phe Asp
       770                 775                 780
   Ala Ile Gln Pro Ser Arg Tyr Val Val Gly Leu Gly Tyr Asp Gln Pro
   785                 790                 795                 800
   Glu Gly Lys Trp Gly Val Asn Gly Met Leu Thr Tyr Ser Lys Ala Lys
                   805                 810                 815
   Glu Ile Thr Glu Leu Leu Gly Ser Arg Ala Leu Leu Asn Gly Asn Ser
               820                 825                 830
   Arg Asn Thr Lys Ala Thr Ala Arg Arg Thr Arg Pro Trp Tyr Ile Val
           835                 840                 845
   Asp Val Ser Gly Tyr Tyr Thr Val Lys Lys His Phe Thr Leu Arg Ala
       850                 855                 860
   Gly Val Tyr Asn Leu Leu Asn Tyr Arg Tyr Val Thr Trp Glu Asn Val
   865                 870                 875                 880
   Arg Gln Thr Ala Gly Gly Ala Val Asn Gln His Lys Asn Val Gly Val
                   885                 890                 895
   Tyr Asn Arg Tyr Ala Ala Pro Gly Arg Asn Tyr Thr Phe Ser Leu Glu
               900                 905                 910
   Met Lys Phe
           915
<210> 24
   <211> 712
   <212> PRT
   <213> Neisseria meningitidis
<400> 24
   Met Asn Asn Pro Leu Val Asn Gln Ala Ala Met Val Leu Pro Val Phe
   1               5                   10                  15
   Leu Leu Ser Ala Cys Leu Gly Gly Gly Gly Ser Phe Asp Leu Asp Ser
               20                  25                  30
   Val Asp Thr Glu Ala Pro Arg Pro Ala Pro Lys Tyr Gln Asp Val Phe
           35                  40                  45
   Ser Glu Lys Pro Gln Ala Gln Lys Asp Gln Gly Gly Tyr Gly Phe Ala
       50                  55                  60
   Met Arg Leu Lys Arg Arg Asn Trp Tyr Pro Gln Ala Lys Glu Asp Glu
   65                  70                  75                  80
   Val Lys Leu Asp Glu Ser Asp Trp Glu Ala Thr Gly Leu Pro Asp Glu
                   85                  90                  95
   Pro Lys Glu Leu Pro Lys Arg Gln Lys Ser Val Ile Glu Lys Val Glu
               100                 105                 110
   Thr Asp Ser Asp Asn Asn Ile Tyr Ser Ser Pro Tyr Leu Lys Pro Ser
           115                 120                 125
   Asn His Gln Asn Gly Asn Thr Gly Asn Gly Ile Asn Gln Pro Lys Asn
       130                 135                 140
   Gln Ala Lys Asp Tyr Glu Asn Phe Lys Tyr Val Tyr Ser Gly Trp Phe
   145                 150                 155                 160
   Tyr Lys His Ala Lys Arg Glu Phe Asn Leu Lys Val Glu Pro Lys Ser
                   165                 170                 175
   Ala Lys Asn Gly Asp Asp Gly Tyr Ile Phe Tyr His Gly Lys Glu Pro
               180                 185                 190
   Ser Arg Gln Leu Pro Ala Ser Gly Lys Ile Thr Tyr Lys Gly Val Trp
           195                 200                 205
   His Phe Ala Thr Asp Thr Lys Lys Gly Gln Lys Phe Arg Glu Ile Ile
       210                 215                 220
   Gln Pro Ser Lys Ser Gln Gly Asp Arg Tyr Ser Gly Phe Ser Gly Asp
   225                 230                 235                 240
   Asp Gly Glu Glu Tyr Ser Asn Lys Asn Lys Ser Thr Leu Thr Asp Gly
                   245                 250                 255
   Gln Glu Gly Tyr Gly Phe Thr Ser Asn Leu Glu Val Asp Phe His Asn
               260                 265                 270
   Lys Lys Leu Thr Gly Lys Leu Ile Arg Asn Asn Ala Asn Thr Asp Asn
           275                 280                 285
   Asn Gln Ala Thr Thr Thr Gln Tyr Tyr Ser Leu Glu Ala Gln Val Thr
       290                 295                 300
   Gly Asn Arg Phe Asn Gly Lys Ala Thr Ala Thr Asp Lys Pro Gln Gln
   305                 310                 315                 320
   Asn Ser Glu Thr Lys Glu His Pro Phe Val Ser Asp Ser Ser Ser Leu
                   325                 330                 335
   Ser Gly Gly Phe Phe Gly Pro Gln Gly Glu Glu Leu Gly Phe Arg Phe
               340                 345                 350
   Leu Ser Asp Asp Gln Lys Val Ala Val Val Gly Ser Ala Lys Thr Lys
           355                 360                 365
   Asp Lys Pro Ala Asn Gly Asn Thr Ala Ala Ala Ser Gly Gly Thr Asp
       370                 375                 380
   Ala Ala Ala Ser Asn Gly Ala Ala Gly Thr Ser Ser Glu Asn Gly Lys
   385                 390                 395                 400
   Leu Thr Thr Val Leu Asp Ala Val Glu Leu Lys Leu Gly Asp Lys Glu
                   405                 410                 415
   Val Gln Lys Leu Asp Asn Phe Ser Asn Ala Ala Gln Leu Val Val Asp
               420                 425                 430
   Gly Ile Met Ile Pro Leu Leu Pro Glu Ala Ser Glu Ser Gly Asn Asn
           435                 440                 445
   Gln Ala Asn Gln Gly Thr Asn Gly Gly Thr Ala Phe Thr Arg Lys Phe
       450                 455                 460
   Asp His Thr Pro Glu Ser Asp Lys Lys Asp Ala Gln Ala Gly Thr Gln
   465                 470                 475                 480
   Thr Asn Gly Ala Gln Thr Ala Ser Asn Thr Ala Gly Asp Thr Asn Gly
                   485                 490                 495
   Lys Thr Lys Thr Tyr Glu Val Glu Val Cys Cys Ser Asn Leu Asn Tyr
               500                 505                 510
   Leu Lys Tyr Gly Met Leu Thr Arg Lys Asn Ser Lys Ser Ala Met Gln
           515                 520                 525
   Ala Gly Glu Ser Ser Ser Gln Ala Asp Ala Lys Thr Glu Gln Val Glu
       530                 535                 540
   Gln Ser Met Phe Leu Gln Gly Glu Arg Thr Asp Glu Lys Glu Ile Pro
   545                 550                 555                 560
   Ser Glu Gln Asn Ile Val Tyr Arg Gly Ser Trp Tyr Gly Tyr Ile Ala
                   565                 570                 575
   Asn Asp Lys Ser Thr Ser Trp Ser Gly Asn Ala Ser Asn Ala Thr Ser
               580                 585                 590
   Gly Asn Arg Ala Glu Phe Thr Val Asn Phe Ala Asp Lys Lys Ile Thr
           595                 600                 605
   Gly Thr Leu Thr Ala Asp Asn Arg Gln Glu Ala Thr Phe Thr Ile Asp
       610                 615                 620
   Gly Asn Ile Lys Asp Asn Gly Phe Glu Gly Thr Ala Lys Thr Ala Glu
   625                 630                 635                 640
   Ser Gly Phe Asp Leu Asp Gln Ser Asn Thr Thr Arg Thr Pro Lys Ala
                   645                 650                 655
   Tyr Ile Thr Asp Ala Lys Val Gln Gly Gly Phe Tyr Gly Pro Lys Ala
               660                 665                 670
   Glu Glu Leu Gly Gly Trp Phe Ala Tyr Pro Gly Asp Lys Gln Thr Lys
           675                 680                 685
   Asn Ala Thr Asn Ala Ser Gly Asn Ser Ser Ala Thr Val Val Phe Gly
       690                 695                 700
   Ala Lys Arg Gln Gln Pro Val Arg
   705                 710
<210> 25
   <211> 186
   <212> PRT
   <213> Neisseria meningitidis
<400> 25
   Met Asn Met Lys Thr Leu Leu Ala Leu Ala Val Ser Ala Val Cys Ser
   1               5                   10                  15
   Val Gly Val Ala Gln Ala His Glu His Asn Thr Ile Pro Lys Gly Ala
               20                  25                  30
   Ser Ile Glu Val Lys Val Gln Gln Leu Asp Pro Val Asn Gly Asn Lys
           35                  40                  45
   Asp Val Gly Thr Val Thr Ile Thr Glu Ser Asn Tyr Gly Leu Val Phe
       50                  55                  60
   Thr Pro Asp Leu Gln Gly Leu Ser Glu Gly Leu His Gly Phe His Ile
   65                  70                  75                  80
   His Glu Asn Pro Ser Cys Glu Pro Lys Glu Lys Glu Gly Lys Leu Thr
                   85                  90                  95
   Ala Gly Leu Gly Ala Gly Gly His Trp Asp Pro Lys Gly Ala Lys Gln
               100                 105                 110
   His Gly Tyr Pro Trp Gln Asp Asp Ala His Leu Gly Asp Leu Pro Ala
           115                 120                 125
   Leu Thr Val Leu His Asp Gly Thr Ala Thr Asn Pro Val Leu Ala Pro
       130                 135                 140
   Arg Leu Lys His Leu Asp Asp Val Arg Gly His Ser Ile Met Ile His
   145                 150                 155                 160
   Thr Gly Gly Asp Asn His Ser Asp His Pro Ala Pro Leu Gly Gly Gly
                   165                 170                 175
   Gly Pro Arg Met Ala Cys Gly Val Ile Lys
               180                 185
<210> 26
   <211> 16
   <212> DNA
   <213> Neisseria meningitidis
<400> 26
   tttgcggggg gggggg 16
<210> 27
   <211> 16
   <212> DNA
   <213> Neisseria meningitidis
<400> 27
   tttgcgaggg aggtgg 16
<210> 28
   <211> 17
   <212> DNA
   <213> Neisseria meningitidis
<400> 28
   ttttgcgagg gaggtgg 17
<210> 29
   <211> 16
   <212> DNA
   <213> Neisseria meningitidis
<400> 29
   ggcggaagga atactt 16
<210> 30
   <211> 35
   <212> DNA
   <213> Neisseria meningitidis
<400> 30
   ttgacatttg cgagggaggt ggtataattg aagac 35
<210> 31
   <211> 40
   <212> DNA
   <213> Neisseria meningitidis
<400> 31
   aaaaatggtt ttttgcgggg gggggggtat aattgaagac 40
<210> 32
   <211> 40
   <212> DNA
   <213> Artificial Sequence
<220>
   <223> Modified porA promoter
<400> 32
   aaaaatggtt ttttgcgagg gaggtggtat aattgaagac 40
<210> 33
   <211> 40
   <212> DNA
   <213> Neisseria meningitidis
<400> 33
   aaaaattgac atttgcgagg gaggtggtat aattgaagac 40
<210> 34
   <211> 40
   <212> DNA
   <213> Neisseria meningitidis
<400> 34
   aaaattgaca ttttgcgagg gaggtggtat aattgaagac 40
<210> 35
   <211> 40
   <212> DNA
   <213> Artificial Sequence
<220>
   <223> Hybrid rRNA promoter
<400> 35
   atatcttgac aggcggaagg aatactttat aattcgcaac 40
<210> 36
   <211> 39
   <212> DNA
   <213> Artificial Sequence
<220>
   <223> Hybrid rRNA promoter
<400> 36
   atatcttgac aggcggaagg aatactttat attcgcaac 39
<210> 37
   <211> 38
   <212> DNA
   <213> Artificial Sequence
<220>
   <223> Hybrid rRNA promoter
<400> 37
   atatcttgac aggcggaagg aatactttta attcgcac 38
<210> 38
   <211> 39
   <212> DNA
   <213> Artificial Sequence
<220>
   <223> Hybrid rRNA promoter
<400> 38
   atatcttgac aggcggaagg aaactttata attcgcaac 39
<210> 39
   <211> 56
   <212> DNA
   <213> Artificial Sequence
<220>
   <223> Hybrid rRNA promoter
<400> 39
   gtatcgggtg tttgcccgat gtttttaggt ttttatcaaa tttacaaaag gaagcc 56
<210> 40
   <211> 42
   <212> DNA
   <213> Artificial Sequence
<220>
   <223> Hybrid rRNA promoter
<400> 40
   aaaaatggtt ttttgcgggg gggggggggt ataattgaag ac 42
<210> 41
   <211> 358
   <212> DNA
   <213> Artificial Sequence
<220>
   <223> Promoter region
<400> 41
   cgtctgagtc cccgagtttc agacagcata ttcacaaagg cgcaccagcc ggaggaggga  60
   gaggaaagga ttgttggagg cggcgcagta tttagcagaa ataaaaaacc ttatccgaca  120
   gcgacatgac gaatttcccc aaaaaaatcc cgctgaaagc attgaccgtt tttccctgtg  180
   ggcgtatagt tcggttcttc gctgctgcag aagtggcgga cgaactgaaa agtatagcac  240
   agaatgttgg ggatatcgag agatatcttg acaggcggaa ggaatacttt ataattcgca  300
   acgtatcggg tgtttgcccg atgtttttag gtttttatca aatttacaaa aggaagcc    358
<210> 42
   <211> 356
   <212> DNA
   <213> Artificial Sequence
<220>
   <223> Promoter region
<220>
   <221> misc_feature
   <222> 318, 319, 321
   <223> 'n' is a, c, g or t
<400> 42
   cgtctgagtc cccgagtttc agacagcata ttcacaaagg cgcaccagcc ggaggaggga  60
   gaggaaagga ttgttggagg cggcgcagta tttagcagaa ataaaaaacc ttatccgaca  120
   gcgacatgac gaatttcccc aaaaaaatcc cgctgaaagc attgaccgtt tttccctgtg  180
   ggcgtatagt tcggttcttc gctgctgcag aagtggcgga cgaactgaaa agtatagcac  240
   agaatgttgg ggatatcgag agatatcttg acaggcggaa ggaatacttt atattcgcaa  300
   cgtatcgggt gtttgccnna ngtttttagg tttttatcaa atttcaaaag gaagcc      356
<210> 43
   <211> 373
   <212> DNA
   <213> Artificial Sequence
<220>
   <223> Promoter region
<220>
   <221> misc_feature
   <222> 361
   <223> 'n' is a, c, g or t
<400> 43
   cgtctgagtc cccgagtttc agacagcata ttcacaaagg cgcaccagcc ggaggaggga  60
   gaggaaagga ttgttggagg cggcgcagta tttagcagaa ataaaaaacc ttatccgaca  120
   gcgacatgac gaatttcccc aaaaaaatcc cgctgaaagc attgaccgtt tttccctgtg  180
   ggcgtatagt tcggttcttc gctgctgcag aagtggcgga cgaactgaaa agtatagcac  240
   agaatgttgg ggatatcgag agatatcttg acaggcggaa ggaatacttt taattcgcac  300
   gtatcgggtg tttgcccgat gtttttaggt ttttattaaa tttacaaaag gaagcccata  360
   ngaatcgaac tgc                                                     373